# EUROPEAN PATENT APPLICATION

(11) **EP 4 060 335 A1**
(43) Date of publication of application: **21.09.2022**
(21) Application number: 20897108.5
(22) Date of filing: 04.12.2020
(51) Int. Cl.: G01N 33/15, G01N 33/38, G01N 33/50, G01N 33/574

(54) **METHOD FOR DIAGNOSING CANCER, CANCER DIAGNOSIS COMPOSITION, CANCER DIAGNOSIS KIT, METHOD FOR EVALUATING STATE OF CANCER, AND METHOD FOR SCREENING AGENTS FOR PREVENTING AND/OR TREATING CANCER**

(30) Priority: 05.12.2019 JP 2019220139
(71) Applicant: Kyushu University, National University Corporation, Nishi-ku Fukuoka-shi Fukuoka 819-0395 (JP)
(72) Inventor: MURATA Masaharu, Fukuoka-shi, Fukuoka 819-0395 (JP); KAWANO Takahito, Fukuoka-shi, Fukuoka 819-0395 (JP); ETO Masatoshi, Fukuoka-shi, Fukuoka 819-0395 (JP); INOKUCHI Junichi, Fukuoka-shi, Fukuoka 819-0395 (JP); KANG Jeong-Hun, Kishibe-Shimmachi, Suita-shi, Osaka 564-8565 (JP)
(74) Representative: Hoffmann Eitle
(86) International application number: PCT/JP2020/045257
(87) International publication number: WO 2021/112225

(57) **Abstract**

The present invention discloses a method for diagnosing cancer comprising a step of detecting active protein kinase Cα in urine.

SEQUENCE LISTING

<110>  KYUSHU UNIVERSITY, NATIONAL UNIVERSITY CORPORATION

<120>  METHOD FOR DIAGNOSING CANCER, COMPOSITION FOR DIAGNOSING CANCER,
       KIT DIAGNOSING CANCER,  METHOD OF EVALUATING CONDITION OF CANCER,
        AND DRUG FOR PREVENTING AND/OR TREATING CANCER

<130>  FP20-1113-00

<150>  JP2019-220139
<151>  2019-12-05

<160>  12

<170>  PatentIn version 3.5

<210>  1
<211>  11
<212>  PRT
<213>  Artificial Sequence

<220>
<223>  Synthetic peptide

<400>  1

Phe Lys Lys Gln Gly Ser Phe Ala Lys Lys Lys
1               5                   10


<210>  2
<211>  11
<212>  PRT
<213>  Artificial Sequence

<220>
<223>  Synthetic peptide

<400>  2

Phe Lys Lys Gln Gly Thr Phe Ala Lys Lys Lys
1               5                   10


<210>  3
<211>  8964
<212>  DNA
<213>  Homo sapiens

<400>  3
gtcagtgagc ctggggccag cgggcgagcc agcggctccg gctcccgctc ccgctccgcg      60

cagcaccagc ccgactctcc ccggcccccg ccgcgccccc tcgccgcgac ctcggccacc      120

ggcccgcgcc ccgcgcccgg ggtcgccccg agcccgcacc tctcccccgc cgcccccgcc      180

cacccggccc tccgcggccg cagctccccg gcggaggcaa gaggtggttg ggggggacca      240

tggctgacgt tttcccgggc aacgactcca cggcgtctca ggacgtggcc aaccgcttcg      300

cccgcaaagg ggcgctgagg cagaagaacg tgcacgaggt gaaggaccac aaattcatcg      360

cgcgcttctt caagcagccc accttctgca gccactgcac cgacttcatc tgggggtttg 420

ggaaacaagg cttccagtgc caagtttgct gttttgtggt ccacaagagg tgccatgaat 480

ttgttacttt ttcttgtccg ggtgcggata agggacccga cactgatgac cccaggagca 540

agcacaagtt caaaatccac acttacggaa gccccacctt ctgcgatcac tgtgggtcac 600

tgctctatgg acttatccat caagggatga aatgtgacac ctgcgatatg aacgttcaca 660

agcaatgcgt catcaatgtc cccagcctct gcggaatgga tcacactgag aagagggggc 720

ggatttacct aaaggctgag gttgctgatg aaaagctcca tgtcacagta cgagatgcaa 780

aaaatctaat ccctatggat ccaaacgggc tttcagatcc ttatgtgaag ctgaaactta 840

ttcctgatcc caagaatgaa agcaagcaaa aaaccaaaac catccgctcc acactaaatc 900

cgcagtggaa tgagtccttt acattcaaat tgaaaccttc agacaaagac cgacgactgt 960

ctgtagaaat ctgggactgg gatcgaacaa caaggaatga cttcatggga tccctttcct 1020

ttggagtttc ggagctgatg aagatgccgg ccagtggatg gtacaagttg cttaaccaag 1080

aagaaggtga gtactacaac gtacccattc cggaagggga cgaggaagga aacatggaac 1140

tcaggcagaa attcgagaaa gccaaacttg gccctgctgg caacaaagtc atcagtccct 1200

ctgaagacag gaaacaacct tccaacaacc ttgaccgagt gaaactcacg gacttcaatt 1260

tcctcatggt gttgggaaag gggagttttg gaaaggtgat gcttgccgac aggaagggca 1320

cagaagaact gtatgcaatc aaaatcctga agaaggatgt ggtgattcag gatgatgacg 1380

tggagtgcac catggtagaa aagcgagtct tggccctgct tgacaaaccc ccgttcttga 1440

cgcagctgca ctcctgcttc cagacagtgg atcggctgta cttcgtcatg gaatatgtca 1500

acggtgggga cctcatgtac cacattcagc aagtaggaaa atttaaggaa ccacaagcag 1560

tattctatgc ggcagagatt tccatcggat tgttctttct tcataaaaga ggaatcattt 1620

atagggatct gaagttagat aacgtcatgt tggattcaga aggacatatc aaaattgctg 1680

actttgggat gtgcaaggaa cacatgatgg atggagtcac gaccaggacc ttctgtggga 1740

ctccagatta tatcgcccca gagataatcg cttatcagcc gtatggaaaa tctgtggact 1800

ggtgggccta tggcgtcctg ttgtatgaaa tgcttgccgg gcagcctcca tttgatggtg 1860

aagatgaaga cgagctattt cagtctatca tggagcacaa cgtttcctat ccaaaatcct 1920

tgtccaagga ggctgtttct gtctgcaaag gactgatgac caaacaccca gccaagcggc 1980

tgggctgtgg gcctgagggg gagagggacg tgagagagca tgccttcttc cggaggatcg 2040

actgggaaaa actggagaac agggagatcc agccaccatt caagcccaaa gtgtgtggca 2100

aaggagcaga gaactttgac aagttcttca cacgaggaca gcccgtctta acaccacctg 2160

atcagctggt tattgctaac atagaccagt ctgattttga agggttctcg tatgtcaacc 2220

cccagtttgt gcaccccatc ttacagagtg cagtatgaaa ctcaccagcg agaacaaaca 2280

cctccccagc ccccagccct ccccgcagtg ggaagtgaat ccttaaccct aaaattttaa 2340

ggccacggcc ttgtgtctga ttccatatgg aggcctgaaa attgtagggt tattagtcca 2400

aatgtgatca actgttcagg gtctctctct tacaaccaag aacattatct tagtggaaga 2460

tggtacgtca tgctcagtgt ccagtttaat tctgtagaag ttacgtctgg ctctaggtta 2520

acccttccta gaaagcaagc agactgttgc cccattttgg gtacaatttg atatactttc 2580

cataccctcc atctgtggat ttttcagcat tggaatcccc caaccagaga tgttaaagtg 2640

agcctgtccc aggaaacatc tccacccaag acgtctttgg aatccaagaa caggaagcca 2700

agagagtgag cagggaggga ttgggggtgg gggaggcctc aaaataccga ctgcgtccat 2760

tctctgcctc catggaaaca gcccctagaa tctgaaaggc cgggataaac ctaatcactg 2820

ttcccaaaca ttgacaaatc ctaacccaac catggtccag cagttaccag tttaaacaaa 2880

aaaacctcag atgagtgttg ggtgaatctg tcatctggta ccctccttgg ttgataactg 2940

tcttgatact tttcattctt tgtaagaggc caaatcgtct aaggacgttg ctgaacaagc 3000

gtgtgaaatc atttcagatc aaggataagc cagtgtgtac atatgttcat tttaatctct 3060

gggagattat ttttccatcc agggtgccat cagtaatcat gccactactc accagtgttg 3120

ttcaccaaca cccaccccca cacacaccaa cattttgctg cctaccttgt tatccttctc 3180

aagaagctga agtgtacgcc ctctcccctt ttgtgcttat ttatttaata ggctgcagtg 3240

tcgcttatga aagtacgatg tacagtaact taatggaagt gctgactcta gcatcagcct 3300

ctaccgattg attttcctcc cttctctagc cctggatgtc cacttaggga taaaaagaat 3360

atggttttgg ttcccatttc tagttcacgt tgaatgacag gcctggagct gtagaatcag 3420

gaaacccgga tgcctaacag ctcaaagatg ttttgttaat agaaggattt taatacgttt 3480

tgcaaatgca tcatgcaatg aattttgcat gtttataata aaccttaata acaagtgaat 3540

ctatattatt gatataatcg tatcaagtat aaagagagta ttataataat tttataagac 3600

acaattgtgc tctatttgtg caggttcttg tttctaatcc tcttttctaa ttaagtttta 3660

gctgaatccc ttgcttctgt gctttccctc cctgcacatg ggcactgtat cagatagatt 3720

actttttaaa tgtagataaa atttcaaaaa tgaatggcta gtttacgtga tagattaggc 3780

tcttactaca tatgtgtgtg tatatatatg tatttgattc tacctgcaaa caaattttta 3840

ttggtgagga ctatttttga gctgacactc cctcttagtt tcttcatgtc acctttcgtc 3900

ctggttcctc cgccactctt cctcttgggg acaacaggaa gtgtctgatt ccagtctgcc 3960

tagtacgttg gtacacacgt ggcattgccg cagcacctgg gctgaccttt gtgtgtgcgt 4020

gtgtgtgtgt ttccttcttc ccttcagcct gtgactgttg ctgactccag ggtgggaggg 4080

gatggggaga ctcccctctt gctgtgtgta ctggacacgc aggaagcatg ctgtcttgct 4140

gcctctgcaa cgacctgtcg tttgctccag catgcacaaa cttcgtgaga ccaacacagc 4200

cgtgccctgc aggcaccagc acgtgctttt cagaggctgc ggactttctt ccagccattg 4260

tggcattggc ctttccagtc ttgggaggag cgcgctgctt tggtgagaca cccccatgca 4320

aggtcctcag agtagccggg ttctaccaca aacagaaaca gaatgaaagt agctgtcagt 4380

ccttgtagag agccgctctg tttcctccca gaagcatctc ccagctaagc tcgcattatt 4440

tttctcctct ggctgtttgc ctgaagttca cagaacacac aaccatgaaa ggctttttga 4500

ggtgagaggc ccaggtggtc ctggcaaccc tgagtagaag gagagacggg gtagggaacg 4560

ggcccggcca gaaaagaacc atttcttctg ccatctttta tgcaccatag acatcgagac 4620

tccagggggt cctggctccc ctgtccctgc agccctgcag gtcagtgcat gatctgggtt 4680

cgtgtcctga ccaggtgctc ctcctttgat ccgaggggaa agggactggt ttatagaaag 4740

agcctaggag acaaaagggc cagtccccct gcccagaatg gagcagcagc aggacagacc 4800

cccacgaggc cccccagaga ggaggaagat cccacggagg aacacatgag gttagggacc 4860

cttgttcagc accccaaaca gcctgcctgt ttaaagcagg cagcaggctt aggccttccc 4920

tgcaacccca acacccacaa gtttgtttct ctaggaaaca cattcactgt ctcagctggc 4980

tgttactctc tcagaccata tggcaaagtt ttccaagaaa atgccccgac aggggtgccc 5040

agcacactgc ctgagggaca acagacatca gaacaaaccc ccagagagaa acagtcaaaa 5100

tcagggcccg gtgcagtgtt gtcatgtgga acctgcttta tccattgctg agtgttgaat 5160

gtgggtaatg gttagggctt tccagatctc agcagccaaa gacagttatt gttggaagac 5220

tgtcatgtag ataaccatga gcaatggctc gcctcagaat cagttcataa aattctatgg 5280

tactggcccc ttcgtgggta ttgtgtgaaa tgagatggtg gcgaggggtg cgctgtggaa 5340

ctgccgcagc cacgcaggag gtccctgggg gatgctttgg gaagtccttg cccctgagca 5400

ctgcctgatt gccagggcct gtggaggtct aggccgcctg gcagaatcta gcaccgtccg 5460

aatccccgca ggacccatgg agctatgacc acaccaggcc attcaaatgg ctctgcatta 5520

tcttcccttg gaaggtggcc actcctcggt ggcagggcct ttccctgagg ctgcaggccg 5580

tgggctggca gcccgtctct tggcatttca attgaaggtc accaggtgct gggtttgaaa 5640

ggaagtcact ggagtgctgc caggggccgc cctccaaggt taatgagagg cccacatcca 5700

ggcaagaact aattcaaaag gcagatcaga aaccacagga gtcaaaatta ttgctccggc 5760

agtgcttccc ttcctttcat ccactggcct cgtgtggtcc atgcagggcc actgtctgcc 5820

ctttctgatg ccacgtatta ggctttctta ctcagaattt tgatagaaaa ccatggggcc 5880

aagagctctg gaagcctggc cggaaagacc aaggttcatg cagcccaaca aatgattgtt 5940

gagcacctct cggagccaaa gtccttaggc gagtgtggtg acttcctgga aggaggatgc 6000

agacttccag agagcccccc caacggacgt gctgagaagg gagagggagg cgggggctgt 6060

agtcaggaag gagccagaga agaacagggt ttgggtgcat ccagaaatat gcctgcagta 6120

ggagggagag gaaggggtgc caccgtcaac ggcttcccat cggaggtggt tggtgcagat 6180

ggaagtttct gtctgctggc cctcaagaga gtgttttgcc agggacacag tctgttcctc 6240

ctcagaaaac accccccaaa tgctaacaac atccccacca gctgctagaa gcccctttcc 6300

cctccccacc ttgaagtagc tcatagttct ctgggcagag ccagaccatc cagtgtaccc 6360

cagaggccag taggttcctg cccattttcc tctctggctt cctgccaaga attatggcag 6420

ctgaggatga atggagaagt aaaaacaact aacaccgcac aactaacaac taacaccgca 6480

gttcccacct gggttccact tagcaggaga catttcggag ggtttttttt gtttttgttc 6540

ctgttttttt tttttttgct ggaatttgtt ttctcagtac tgaaaagaga aaaagtgaca 6600

atcttgtatt tttaaaagcc tcggaaaggt gataccatct gacagtcatt ttctcacgtt 6660

ggtcttctaa agtcacctat ttcttgtgtg tgcacatcac accatttcct gtttctttat 6720

aacccgacaa gggtaggagt gcctgtttcc cctgctgggc acaccagaca atcgtaatca 6780

caaaacagac actgagccag gggcccaaag ggtgtgatca tgagagttac cgggacagca 6840

gtaggcatga cagtcaccag gaaggacaag ggtgctctgt tgttagtggc cacacaccaa 6900

tttgacaagg agtgttgcga aatttttatt tatttattta tttattttga gatggagttt 6960

cactcttgtt gcccaggctg gagtgcggtg gtacaatctc ggctcactgc aacctccacc 7020

tcccaggttc aagcgattct cctgcctcag cctcccaagt acctgggact acaggtgcgt 7080

gccaccacac ccagctaaat tttgtgtttt tagtagagat ggggtttcac catgttggcc 7140

aggatggtct tgaacccctg acctcatgat ctgcctgcct cggcctccca aagtgctggg 7200

attacaggca tgagccacca cgcccagcca aaatattttt ttaaagtcat tttccttaag 7260

ctgcttgggc tacatgtgaa atacactgga cggtcaacat tcctgtctcc tcccatttgg 7320

gctgatgcag cagatccagg gaatgttacc tgtttctgct gctagaagat ccaggaaatt 7380

gggaaggtta cctgacgcac acatggatga aggccatcat ctagaaatgg ggtcaaccac 7440

aattgtgtta attccgtagt gtcagggatt cttcgggaag gtcaacagta tgaaggattc 7500

tgacccctgt gcctcccatt tatgtgatca ggtgacagtt aataaccgtg gaggtcacac 7560

tcagccatcc aacagcctta cagtgaccct acacaaaagc ccccaaattc caaagacttt 7620

ttcttaacct aaaggaagaa attatttgtt aattccagta gagcaactga atatactggg 7680

ctatttgtac ttttttatag agaactttaa taataattct ttaaaaatga gtttttagaa 7740

caaagcaact gacgatttcc taagattcca atgccctgga gcttgtagga ggacttagcc 7800

tgggtcagct ggagcacccc cgacctgatc tcccactgcc agattttccc atgctcctag 7860

ggtatggagt ccacgtggga atgactgcaa gttcaggtgg aacttggccg actgatgctc 7920

tgcgagtttt taatagacac tggggacaac tgcttaaggt ttagaaactt ccaaaccaca 7980

ggaaagacat ttttagtgtc ccccatccag aggcagccct ggaataggat tcccaggggt 8040

ttctgggacc cctttccttg ctccgtgagg ctctgtggcc atcttttggc aggaggagga 8100

tgcttccttg gctctgtgcc cagacccgcc tggtccccag gtctctcacc ttgggtgaag 8160

attcagagat gccctgtaag gattttgccc actgggcaac tcagaaatac ttcgatctcc 8220

caagatataa gaggcagcag caaacgtgcc tattgacgtc tgtttcatag ttaccactta 8280

cgcgagtaga cagaactcgg cttttcagaa aataggtgtc aagtccactt tataagaacc 8340

tttttttcta aaataagata aaaggtggct ttgcattttc tgattaaacg actgtgtctt 8400

tgtcacctct gcttaacttt aggagtatcc attcctgtga ttgtagactt ttgttgatat 8460

tcttcctgga agaatatcat tcttttcttg aagggttggt ttactagaat attcaaaatc 8520

aatcatgaag gcagttacta ttttgagtct aaaggttttc taaaaattaa cctcacatcc 8580

cttctgttag ggtctttcag aatatctttt ataaacagaa gcatttgaag tcattgcttt 8640

tgctacatga tttgtgtgtg tgaaggacat accacgttta aatcattaat tgaaaaacat 8700

catataagcc ccaactttgt ttggaggaag agacggaggt tgaggttttt ccttctgtat 8760

aagcacctac tgacaaaatg tagaggccat tcaaccgtca aacaccattt ggttatatcg 8820

cagaggagac ggatgtgtaa attactgcat tgcttttttt ttcagtttgt ataacctcta 8880

atctccgttt gcatgatacg ctttgttaga aacattaatt gtagtttgga agcaagtgtg 8940

tatgaataaa gataatgatc attc 8964

<210> 4
<211> 672
<212> PRT
<213> Homo sapiens

<400> 4

Met Ala Asp Val Phe Pro Gly Asn Asp Ser Thr Ala Ser Gln Asp Val
1               5                   10                  15

Ala Asn Arg Phe Ala Arg Lys Gly Ala Leu Arg Gln Lys Asn Val His
            20                  25                  30

Glu Val Lys Asp His Lys Phe Ile Ala Arg Phe Phe Lys Gln Pro Thr
        35                  40                  45

Phe Cys Ser His Cys Thr Asp Phe Ile Trp Gly Phe Gly Lys Gln Gly
    50                  55                  60

Phe Gln Cys Gln Val Cys Cys Phe Val Val His Lys Arg Cys His Glu
65                  70                  75                  80

Phe Val Thr Phe Ser Cys Pro Gly Ala Asp Lys Gly Pro Asp Thr Asp
                85                  90                  95

Asp Pro Arg Ser Lys His Lys Phe Lys Ile His Thr Tyr Gly Ser Pro
            100                 105                 110

Thr Phe Cys Asp His Cys Gly Ser Leu Leu Tyr Gly Leu Ile His Gln
        115                 120                 125

Gly Met Lys Cys Asp Thr Cys Asp Met Asn Val His Lys Gln Cys Val
    130                 135                 140

Ile Asn Val Pro Ser Leu Cys Gly Met Asp His Thr Glu Lys Arg Gly
145                 150                 155                 160

Arg Ile Tyr Leu Lys Ala Glu Val Ala Asp Glu Lys Leu His Val Thr
                165                 170                 175

Val Arg Asp Ala Lys Asn Leu Ile Pro Met Asp Pro Asn Gly Leu Ser
            180                 185                 190

Asp Pro Tyr Val Lys Leu Lys Leu Ile Pro Asp Pro Lys Asn Glu Ser
        195                 200                 205

Lys Gln Lys Thr Lys Thr Ile Arg Ser Thr Leu Asn Pro Gln Trp Asn
    210                 215                 220

Glu Ser Phe Thr Phe Lys Leu Lys Pro Ser Asp Lys Asp Arg Arg Leu
225                 230                 235                 240

Ser Val Glu Ile Trp Asp Trp Asp Arg Thr Thr Arg Asn Asp Phe Met
                245                 250                 255

Gly Ser Leu Ser Phe Gly Val Ser Glu Leu Met Lys Met Pro Ala Ser
            260                 265                 270

Gly Trp Tyr Lys Leu Leu Asn Gln Glu Glu Gly Glu Tyr Tyr Asn Val
        275                 280                 285

Pro Ile Pro Glu Gly Asp Glu Glu Gly Asn Met Glu Leu Arg Gln Lys
    290                 295                 300

Phe Glu Lys Ala Lys Leu Gly Pro Ala Gly Asn Lys Val Ile Ser Pro
305                 310                 315                 320

Ser Glu Asp Arg Lys Gln Pro Ser Asn Asn Leu Asp Arg Val Lys Leu
                325                 330                 335

Thr Asp Phe Asn Phe Leu Met Val Leu Gly Lys Gly Ser Phe Gly Lys
        340                 345                 350

Val Met Leu Ala Asp Arg Lys Gly Thr Glu Glu Leu Tyr Ala Ile Lys
    355                 360                 365

Ile Leu Lys Lys Asp Val Val Ile Gln Asp Asp Asp Val Glu Cys Thr
    370                 375                 380

Met Val Glu Lys Arg Val Leu Ala Leu Leu Asp Lys Pro Pro Phe Leu
385                 390                 395                 400

Thr Gln Leu His Ser Cys Phe Gln Thr Val Asp Arg Leu Tyr Phe Val
                405                 410                 415

Met Glu Tyr Val Asn Gly Gly Asp Leu Met Tyr His Ile Gln Gln Val
            420                 425                 430

Gly Lys Phe Lys Glu Pro Gln Ala Val Phe Tyr Ala Ala Glu Ile Ser
        435                 440                 445

Ile Gly Leu Phe Phe Leu His Lys Arg Gly Ile Ile Tyr Arg Asp Leu
    450                 455                 460

Lys Leu Asp Asn Val Met Leu Asp Ser Glu Gly His Ile Lys Ile Ala
465                 470                 475                 480

Asp Phe Gly Met Cys Lys Glu His Met Met Asp Gly Val Thr Thr Arg
                485                 490                 495

Thr Phe Cys Gly Thr Pro Asp Tyr Ile Ala Pro Glu Ile Ile Ala Tyr
            500                 505                 510

Gln Pro Tyr Gly Lys Ser Val Asp Trp Trp Ala Tyr Gly Val Leu Leu
        515                 520                 525

Tyr Glu Met Leu Ala Gly Gln Pro Pro Phe Asp Gly Glu Asp Glu Asp
    530                 535                 540

Glu Leu Phe Gln Ser Ile Met Glu His Asn Val Ser Tyr Pro Lys Ser
545                 550                 555                 560

Leu Ser Lys Glu Ala Val Ser Val Cys Lys Gly Leu Met Thr Lys His
                565                 570                 575

Pro Ala Lys Arg Leu Gly Cys Gly Pro Glu Gly Glu Arg Asp Val Arg

580                 585                 590

Glu His Ala Phe Phe Arg Arg Ile Asp Trp Glu Lys Leu Glu Asn Arg
        595                 600                 605

Glu Ile Gln Pro Pro Phe Lys Pro Lys Val Cys Gly Lys Gly Ala Glu
    610                 615                 620

Asn Phe Asp Lys Phe Phe Thr Arg Gly Gln Pro Val Leu Thr Pro Pro
625                 630                 635                 640

Asp Gln Leu Val Ile Ala Asn Ile Asp Gln Ser Asp Phe Glu Gly Phe
                645                 650                 655

Ser Tyr Val Asn Pro Gln Phe Val His Pro Ile Leu Gln Ser Ala Val
            660                 665                 670

<210>  5
<211>  2245
<212>  DNA
<213>  Homo sapiens

<400>  5
ggagcaagag gtggttgggg ggggaccatg gctgacgttt tcccgggcaa cgactccacg      60

gcgtctcagg acgtggccaa ccgcttcgcc cgcaaagggg cgctgaggca gaagaacgtg     120

cacgaggtga aggaccacaa attcatcgcg cgcttcttca agcagcccac cttctgcagc     180

cactgcaccg acttcatctg ggggtttggg aaacaaggct tccagtgcca agtttgctgt     240

tttgtggtcc acaagaggtg ccatgaattt gttacttttt cttgtccggg tgcggataag     300

ggacccgaca ctgatgaccc caggagcaag cacaagttca aaatccacac ttacggaagc     360

cccaccttct gcgatcactg tgggtcactg ctctatggac ttatccatca agggatgaaa     420

tgtgacacct gcgatatgaa cgttcacaag caatgcgtca tcaatgtccc cagcctctgc     480

ggaatggatc acactgagaa gagggggcgg atttacctaa aggctgaggt tgctgatgaa     540

aagctccatg tcacagtacg agatgcaaaa aatctaatcc ctatggatcc aaacgggctt     600

tcagatcctt atgtgaagct gaaacttatt cctgatccca agaatgaaag caagcaaaaa     660

accaaaacca tccgctccac actaaatccg cagtggaatg agtcctttac attcaaattg     720

aaaccttcag acaaagaccg acgactgtct gtagaaatct gggactggga tcgaacaaca     780

aggaatgact tcatgggatc cctttccttt ggagtttcgg agctgatgaa gatgccggcc     840

agtggatggt acaagttgct taaccaagaa gaaggtgagt actacaacgt acccattccg     900

gaaggggacg aggaaggaaa catggaactc aggcagaaat tcgagaaagc caaacttggc     960

cctgctggca acaaagtcat cagtccctct gaagacagga aacaaccttc caacaacctt    1020

gaccgagtga aactcacgga cttcaatttc ctcatggtgt tgggaaaggg gagttttgga 1080

aaggtgatgc ttgccgacag gaagggcaca gaagaactgt atgcaatcaa aatcctgaag 1140

aaggatgtgg tgattcagga tgatgacgtg gagtgcacca tggtagaaaa gcgagtcttg 1200

gccctgcttg acaaaccccc gttcttgacg cagctgcact cctgcttcca gacagtggat 1260

cggctgtact tcgtcatgga atatgtcaac ggtggggacc tcatgtacca cattcagcaa 1320

gtaggaaaat ttaaggaacc acaagcagta ttctatgcgg cagagatttc catcggattg 1380

ttctttcttc ataaaagagg aatcatttat agggatctga agttagataa cgtcatgttg 1440

gattcagaag gacatatcaa aattgctgac tttgggatgt gcaaggaaca catgatggat 1500

ggagtcacga ccaggacctt ctgtgggact ccagattata tcgccccaga gataatcgct 1560

tatcagccgt atggaaaatc tgtggactgg tgggcctatg gcgtcctgtt gtatgaaatg 1620

cttgccgggc agcctccatt tgatggtgaa gatgaagacg agctatttca gtctatcatg 1680

gagcacaacg tttcctatcc aaaatccttg tccaaggagg ctgtttctat ctgcaaagga 1740

ctgatgacca aacacccagc caagcggctg ggctgtgggc ctgaggggga gagggacgtg 1800

agagagcatg ccttcttccg gaggatcgac tgggaaaaac tggagaacag ggagatccag 1860

ccaccattca agcccaaagt gtgtggcaaa ggagcagaga actttgacaa gttcttcaca 1920

cgaggacagc ccgtcttaac accacctgat cagctggtta ttgctaacat agaccagtct 1980

gattttgaag ggttctcgta tgtcaacccc cagtttgtgc accccatctt acagagtgca 2040

gtatgaaact caccagcgag aacaaacacc tccccagccc ccagccctcc ccgcagtgga 2100

agtgaatcct taaccctaaa attttaaggc cacggcttgt gtctgattcc atatggaggc 2160

ctgaaaattg tagggttatt agtccaaatg tgatcaactg ttcagggtct ctctcttaca 2220

accaagaaca ttatcttagt ggaag 2245

<210> 6
<211> 672
<212> PRT
<213> Homo sapiens

<400> 6

Met Ala Asp Val Phe Pro Gly Asn Asp Ser Thr Ala Ser Gln Asp Val
1               5                   10                  15

Ala Asn Arg Phe Ala Arg Lys Gly Ala Leu Arg Gln Lys Asn Val His
            20                  25                  30

Glu Val Lys Asp His Lys Phe Ile Ala Arg Phe Phe Lys Gln Pro Thr
        35                  40                  45

Phe Cys Ser His Cys Thr Asp Phe Ile Trp Gly Phe Gly Lys Gln Gly

50                  55                  60

Phe Gln Cys Gln Val Cys Cys Phe Val Val His Lys Arg Cys His Glu
65                  70                  75                  80

Phe Val Thr Phe Ser Cys Pro Gly Ala Asp Lys Gly Pro Asp Thr Asp
                85                  90                  95

Asp Pro Arg Ser Lys His Lys Phe Lys Ile His Thr Tyr Gly Ser Pro
            100                 105                 110

Thr Phe Cys Asp His Cys Gly Ser Leu Leu Tyr Gly Leu Ile His Gln
        115                 120                 125

Gly Met Lys Cys Asp Thr Cys Asp Met Asn Val His Lys Gln Cys Val
    130                 135                 140

Ile Asn Val Pro Ser Leu Cys Gly Met Asp His Thr Glu Lys Arg Gly
145                 150                 155                 160

Arg Ile Tyr Leu Lys Ala Glu Val Ala Asp Glu Lys Leu His Val Thr
                165                 170                 175

Val Arg Asp Ala Lys Asn Leu Ile Pro Met Asp Pro Asn Gly Leu Ser
            180                 185                 190

Asp Pro Tyr Val Lys Leu Lys Leu Ile Pro Asp Pro Lys Asn Glu Ser
        195                 200                 205

Lys Gln Lys Thr Lys Thr Ile Arg Ser Thr Leu Asn Pro Gln Trp Asn
    210                 215                 220

Glu Ser Phe Thr Phe Lys Leu Lys Pro Ser Asp Lys Asp Arg Arg Leu
225                 230                 235                 240

Ser Val Glu Ile Trp Asp Trp Asp Arg Thr Thr Arg Asn Asp Phe Met
                245                 250                 255

Gly Ser Leu Ser Phe Gly Val Ser Glu Leu Met Lys Met Pro Ala Ser
            260                 265                 270

Gly Trp Tyr Lys Leu Leu Asn Gln Glu Glu Gly Glu Tyr Tyr Asn Val
        275                 280                 285

Pro Ile Pro Glu Gly Asp Glu Glu Gly Asn Met Glu Leu Arg Gln Lys
    290                 295                 300

Phe Glu Lys Ala Lys Leu Gly Pro Ala Gly Asn Lys Val Ile Ser Pro
305                 310                 315                 320

Ser Glu Asp Arg Lys Gln Pro Ser Asn Asn Leu Asp Arg Val Lys Leu
                325                 330                 335

Thr Asp Phe Asn Phe Leu Met Val Leu Gly Lys Gly Ser Phe Gly Lys
            340                 345                 350

Val Met Leu Ala Asp Arg Lys Gly Thr Glu Glu Leu Tyr Ala Ile Lys
        355                 360                 365

Ile Leu Lys Lys Asp Val Val Ile Gln Asp Asp Asp Val Glu Cys Thr
    370                 375                 380

Met Val Glu Lys Arg Val Leu Ala Leu Leu Asp Lys Pro Pro Phe Leu
385                 390                 395                 400

Thr Gln Leu His Ser Cys Phe Gln Thr Val Asp Arg Leu Tyr Phe Val
                405                 410                 415

Met Glu Tyr Val Asn Gly Gly Asp Leu Met Tyr His Ile Gln Gln Val
            420                 425                 430

Gly Lys Phe Lys Glu Pro Gln Ala Val Phe Tyr Ala Ala Glu Ile Ser
        435                 440                 445

Ile Gly Leu Phe Phe Leu His Lys Arg Gly Ile Ile Tyr Arg Asp Leu
    450                 455                 460

Lys Leu Asp Asn Val Met Leu Asp Ser Glu Gly His Ile Lys Ile Ala
465                 470                 475                 480

Asp Phe Gly Met Cys Lys Glu His Met Met Asp Gly Val Thr Thr Arg
                485                 490                 495

Thr Phe Cys Gly Thr Pro Asp Tyr Ile Ala Pro Glu Ile Ile Ala Tyr
            500                 505                 510

Gln Pro Tyr Gly Lys Ser Val Asp Trp Trp Ala Tyr Gly Val Leu Leu
        515                 520                 525

Tyr Glu Met Leu Ala Gly Gln Pro Pro Phe Asp Gly Glu Asp Glu Asp
    530                 535                 540

Glu Leu Phe Gln Ser Ile Met Glu His Asn Val Ser Tyr Pro Lys Ser
545                 550                 555                 560

Leu Ser Lys Glu Ala Val Ser Ile Cys Lys Gly Leu Met Thr Lys His
                565                 570                 575

Pro Ala Lys Arg Leu Gly Cys Gly Pro Glu Gly Glu Arg Asp Val Arg
            580                 585                 590

Glu His Ala Phe Phe Arg Arg Ile Asp Trp Glu Lys Leu Glu Asn Arg
        595                 600                 605

Glu Ile Gln Pro Pro Phe Lys Pro Lys Val Cys Gly Lys Gly Ala Glu
    610                 615                 620

Asn Phe Asp Lys Phe Phe Thr Arg Gly Gln Pro Val Leu Thr Pro Pro
625                 630                 635                 640

Asp Gln Leu Val Ile Ala Asn Ile Asp Gln Ser Asp Phe Glu Gly Phe
                645                 650                 655

Ser Tyr Val Asn Pro Gln Phe Val His Pro Ile Leu Gln Ser Ala Val
            660                 665                 670

<210>  7
<211>  8385
<212>  DNA
<213>  Mus musculus

<400>  7
agtgagcgtg gggccagcca gcgagccaga gagccggaga gagccagaga gagcggctcg      60

gctcccagct ccgagtagcg cagcgcccgc ccggctctcc cgggccaccg ccgccaccac     120

cgcacctcat cagcaccgcc acctcggccg ccgcccccgc ccaccccggc cctccccggc     180

tgctgctccc cggcggaggc aagaggtggt tggggggaac catggctgac gtttacccgg     240

ccaacgactc cacggcgtct caggacgtgg ccaaccgctt cgcccgcaaa ggggcgctga     300

ggcagaagaa cgtgcatgag gtgaaagacc acaaattcat cgcccgcttc ttcaagcaac     360

ccaccttctg cagccactgc accgacttca tctgggggtt tgggaaacaa ggcttccagt     420

gccaagtttg ctgttttgtg gttcataaga ggtgccatga gttcgttacg ttctcttgtc     480

cgggtgcgga taagggacct gacactgacg accccaggag caagcacaag ttcaaaatcc     540

acacatacgg aagccctacc ttctgtgatc actgtgggtc cctgctctat ggacttatcc     600

accaagggat gaaatgtgac acctgcgaca tgaatgttca caagcagtgt gtgatcaatg     660

tccctagcct ctgcggaatg gatcacacag agaagagggg gcggatttat ctgaaggctg     720

aggtcactga tgaaaagctc cacgtcacgg tacgagatgc aaaaaatcta atccctatgg     780

atccaaatgg gctttcggat ccttatgtga agctgaaact tatccctgac cccaagaatg     840

agagcaaaca gaaaaccaaa accatccgct ccacactgaa tcctcagtgg aatgagtcct 900

tcacgttcaa attaaaacct tcagacaaag accggcgact gtctgtagaa atctgggact 960

gggatcggac gactcggaat gacttcatgg gatccctttc ctttggtgtc tcagagctaa 1020

tgaagatgcc ggccagtgga tggtataaac tgctcaacca agaagagggc gaatactaca 1080

atgtgcccat tccagaagga gatgaagaag gcaacatgga actcaggcag aagtttgaga 1140

aagccaagct aggccctgct ggtaacaaag tcatcagccc ttcagaagac agaaagcaac 1200

catccaacaa cctggacaga gtgaaactca cagacttcaa cttcctcatg gtgctgggga 1260

aggggagttt tgggaaggtg atgcttgctg acaggaaggg aacggaggaa ctgtacgcca 1320

tcaagatcct gaagaaggac gtggtgatcc aggacgacga cgtggagtgc accatggtgg 1380

agaagcgcgt gctggccctg ctggacaagc cgccatttct gacacagctg cactcctgct 1440

tccagacagt ggaccggctg tacttcgtca tggaatacgt caacggcggg gacctcatgt 1500

accacattca gcaagtcggg aaatttaagg agccacaagc agtattctac gcagccgaga 1560

tctccatcgg actgttcttc cttcataaaa gagggatcat ttacagggat ctgaagctgg 1620

acaatgtcat gctggactca gaagggcaca tcaaaatcgc cgactttggg atgtgcaagg 1680

aacacatgat ggatggagtc acgaccagga ccttctgtgg gactccggac tacattgccc 1740

cagagataat cgcttaccag ccgtacggga agtctgtaga ttggtgggcg tacggtgtgc 1800

tgctgtacga gatgctagcc gggcagcctc cgtttgatgg tgaagatgaa gatgaactgt 1860

ttcagtctat aatggagcac aacgtgtcct accccaaatc cttgtccaag gaagccgtct 1920

ctatctgcaa aggacttatg accaaacacc ctgccaagcg gctgggctgc gggcccgagg 1980

gagagaggga tgtcagagag catgccttct tcaggagaat cgactgggag aaactggaga 2040

acagggagat ccaaccacca ttcaagccca aagtgtgtgg caaaggagca gaaaactttg 2100

acaagttctt cacgcgagga cagcctgtct taacaccacc agatcagctg gtcattgcta 2160

acatagacca atctgatttt gaagggttct cgtatgtcaa cccccagttt gtgcacccaa 2220

tcttgcaaag tgcagtatga aactcaaaaa tgaaagatca aatgcctccc cagcccccag 2280

catccccagc agtgggaaat gatccttaac cataaaaatt taaggctatg gccttgtatt 2340

ttgttccaca cagaggcctg gaaattctag ggacattagt caatacatga tcaacttttt 2400

tcctcacccc ccaaccaaaa gcattatctt agtggatgat gacataaagt acagagtata 2460

gtttaattat gtagaagtca tgtcagctac aagttgattc ttcctaaaaa aaatcaaagc 2520

gacttggacc ctattttttt tgtacgattt gatatattct ccatactttc catttcttgg 2580

attttcacta tcagaatcga ccagagatag taaagtgaac tcaccctaac tcaaggaaac 2640

tcaaggaaac acttccaccc aagatgtctt tggaacaaaa gaacaggaag tccaaagaga 2700

aagcaaagag aggttggggg ctcatatatt caagaccaca tagcttctat tctccttttc 2760

aatggaaaca gtccctagac tcagagaggg caggatgaac ctgatcactg ttcccagtca 2820

tcatagcaca accatagtgc acagtttaag aaggaaagaa aacttcagac aaatattcac 2880

tgaatctacc atctgtaatc ccctgcttgg ttgataacaa tctctataac tcattctttt 2940

taagaggcca aatcatctaa ggactttgct aaacaagcat gtgaaatcat ttcagatcaa 3000

ggataaacca tgtgtacgtt cattctaatc tctgggagat gactcttcaa tccagggtgc 3060

catcagtaat catgccactg ttcacgagtg ttgttagcca accccacaca cacaatattt 3120

tgctaccttt gtaggtaccc ttcctaagaa gctgaaatgt atgaccccat ccccccccct 3180

ttttttacta tttatttaat aagctgcagt gtcgtttatg gaagtacaat gtatagtaat 3240

ttagtcaaag tattgactag cctcagtccc tctagattga ttttcctcct ttctctcgcc 3300

ccacatccac ttagagatga aaaagagcat agtttgggtt cccattctca gttcctattg 3360

aacggcacat gtggaactgt aggatcaaga tactttatgc tgatatcaac tcaaagatgt 3420

tgtcttgcta gaaggatttt aatatgtttt gcgagtgcat catgcaatgg attttgcatg 3480

tttataataa accttaataa cgagttaatc tatattattg atataattgt atcaactata 3540

aagagtatta tgataatttt tataagacac aattgtgcta tatttgtgac gctcatgttt 3600

ccagtctgct ttaagattaa gtgttagctt tattcattgc tgctgggctc tcacccgcta 3660

ctccacacac tggcactcta tcagatatat taatttttta atgtagatgt aattttaaaa 3720

atgaatggct actttacacg tttaactagg gttttactat atatgtgtac atatgtatat 3780

atatatatat atatatttga ttctacctac aaaccccggt tttgtttgtg gggattattt 3840

tttgagataa gactctgtta acttcttcat gttatcctct gttcaagttc ctttatcacc 3900

ccttctcttg gggacagctg aatatattta attccagtat tcctgatatg ttcatatata 3960

tgtggcattg ttacagcacc taggctgaat tgtgtgtgtg tgtgtgtctg tctgtctgtc 4020

tgtctgtctg tctggtactt ttcagcctat gtctttgggg tctctaggga tgaaggggtg 4080

agagaactcc tttctgtgtg tgcatatgga cacccaaaaa tatgtagtct gtctttctgc 4140

cccagaataa ctcactgtgt acttcagaac acacagaatc cttgagacaa actttatctc 4200

catgcctgtg tagagccatc tctgaagctg ttgccaacag actctcttca gaggcacata 4260

ctgtcttaaa gcggttccta gaacattccg gtctttgaag agcatgcctt tgtccaaatg 4320

cctcctaata caaggtgttt agaagaggca ggcttctgca gcataaagga taagtgaaaa 4380

aggagttcct accagtcctt agagaaggca cctctatcga aatcatctca cagctaggct 4440

ctcctctttg ttattctcca cctatctgct tgacagtcac ataccctgca acccaccaag 4500

agtctttgag atacggagca taggctgttc tgaaaatccc taatcagaag gagatatgga 4560

gtcgagagtg ggccaaacca gaaaagagca gtttcttctg caatctttca tgaattctgg 4620

acttcagacc tcaaaaagtg gggaagggtg tcatatatgg atctcagaag tcaagttcac 4680

catcttggta acctagcctt atcaaaaaca ctgtactcag tccaactgta aaggaactgg 4740

cttatcaatg agccttggaa gtgaaagcat tgctcttccc aggtgggaca gcaacaggaa 4800

gctgaacctc tcaaagtggg ggaaaccaca gagaaggagc acagggttcg tagacaatcc 4860

accctgaggt gccaagctgc ctgactctca tgccagtagc aggcttgggt tcttccttaa 4920

accgccagca tccagctccg agtgattcaa cttctctaga aaatgtgtgc ggttccttgg 4980

atggcttgta cgcttagaga atacccacca acattctcac actggaggag ggaaataaca 5040

gagccacaga ccagcaattg cctaaggaac agaacactgg agaacaaact taggagagat 5100

gataaaaatt taaggacgtg ttcatgatgc tgcctattct atacactgct gagggttaaa 5160

ttcagatgaa ggctagtact ttaaagatct ggacagcaga gacaactttt gctacaaaac 5220

taactcacct ggataaccac tagcagctgt taggcaggat caattgatac tattctacat 5280

ccttgtccca cttcacagac cttgtatgaa acagggctac agggaaacat ctgccatttg 5340

aactgaagca accaagcagg aggccagaac caggagggtg ccttgaaggg tttaatcccc 5400

aggggcaggg gtgggaacta tttagaatgt acctgctctc aggctgtgcc tgcattggca 5460

gggtctgtgg aggagaacct agcacaatcc aagtctccac agggacccat caggccgtga 5520

cccagaaaat ccattgaaat aattttgtat tatattcccc tggaaggtgg ccatttccca 5580

ggagcagctc ttccctgttc caccacaggc cggtgggctg ctagctctgc cttcagtgtg 5640

gcatctaaag ttacctggtg cttgggttga atggaaagag ctggagtatg ctgccaggtg 5700

cagccccagg agttaatggg aggcttaccc caggcagcag ctaattcaga aggcagaaga 5760

gaagccccag gagctaaact gattgccctt caccctccta cctctgtggc ctagtttgag 5820

ctagtagcct tttctttaat ttagtctttt ttattagtaa tttgagaaac tcaggaggcc 5880

cagagttttc cggaagcttg gtctgagatg gtcacgtgaa agattaccag aggccagtat 5940

taaccctcaa atgttgctgc tcacaagctc cagttcttag caaataaatc atggtggctt 6000

gccaacccca aaagagccac tcatcgatgt atcacaaaga ggaggaagca gaagtcaaca 6060

ccattaaaga gcagggtttg agtgcaccca ggaagacatg gaaggtggtg gaaaggttgt 6120

gttaatctgc atgggctttc cactgggggt cattgtgcaa atggaagtta ctgttcatca 6180

gccttcaaag gtgtggcttt ttgccagata caaccttttg ttcgttctca gactctccca 6240

actgtaacaa gtcctttgcc cagagccgag ccagccagca gaccctagaa gcacttagtt 6300

ccatctttat ccctctgata attatggctg ctgagactag gtccagaagt cacgaaaaca 6360

accaacaacg acctgccagc ttctgctcag cagagaaaaa taggaagggt ttgtgcaggg 6420

ctttattttc tcaaacgcct ccacaaggca acaccacctg acagtcattt tctcaccttg 6480

gtcttatgaa atcatctatt tcttgtgtgt acatgtcacg ctgtttcctg tttgtttaca 6540

accctagaag ggtagggtta cctgtcccca tgccacggct gagcacatca gtcactctca 6600

ggagatgcgt gctgagccag ggacctgaaa ggatgctgac catggggggt tggggggaca 6660

atagcaagga aggagacagg tcctctggtt agcagtgggt ccacactaac atgacagagt 6720

gttaggaaac tggttttaaa attgttttcc atgaactgct gttaacaaaa tgtgctggac 6780

agtcaacacc cctgccacat ctcactgggc cagcgtagca aatctagaag aagcagatac 6840

tttctgctgt cagaataatc cagaaaatca ggaaagggtg cttgaggcat gcacagacac 6900

agtgttctag aaacggtgtc taatgctgtc ccagggatga tacagcaatg gttaaaaagt 6960

gtggttggct cagcattcgt gtggtcaggt gtcagtcaat gaacagaaga cacaccaggt 7020

tgttaaacag ccttactgtg accctcctca gaagcccaca agttccaaag acttttttta 7080

acctaaaaca agaaattaac ttattaactc caagagagta cctgggtatc ctgggctatt 7140

tgtacttttt tatagaggac tttaataact caattctttt caagtgagtt tttaaaataa 7200

taaaaatttc ataaatttcc aattccatgc aacttatagg aggatttcac ccaggttgga 7260

tagagcaccc acgatctgca ttcccacagc gggattttcc cgtgctagtg aggtctggag 7320

tccacattgg attggctggc atttcctagc agacactaaa gatagctgtg caaggtttag 7380

ctatcgctca actgtgggga tgacggcttt atagctcccc ttcctgatac agcccccgga 7440

aaggattctt ggggatctac aggaccccac tcttcactgt aagagtcaga gcaggaaggg 7500

ggtgcttctt caactctggc tcaagtcccc tctggtctct cagtctctcg catttgatta 7560

aaatggagag atgccctcta aggattctgc ccactgggca attcaagcct cctaccccga 7620

gaagagacag cgctgagtgt gcctgggcat ctgcgtccat gcccatggat gtgagtagac 7680

agaaccctac ttttgagaaa acgcctatcc agcccacttt gtaagaaccc cctttttcta 7740

aactatgaca aaaggcggct ttgcactttc tgatcaaaca acactgtctt ttgtctcctg 7800

cttaactttt aagactattc tgtgaccgac gtttgtagat tttttttttt gttaatattt 7860

tgcctggaaa aatgtgactc tctccttggg aggtttagta gactaataaa aattatacat 7920

gaaggtggtt gctattttga ggctaaaggt ttttctaaga atcgccctcg tatcctgacc 7980

gtgaaggctt ttcaggatac cttttataga cgatcagatg cattcgagct gtgtttccta 8040

catgatttct gttttgtttt atgaaggaca cgccatgttt aaatctctca ttggaaaaaa 8100

aaaaaaaaaa caccatgcaa aaccccaaat tggtttggag gaaggagtga aggtggaggc 8160

tgttgcccga atgggccttt actgatgagc caggggctga tccactgctc gtttggttac 8220

tgggcaggtg aggtagacat gtaaattact gcctagcttt cttggatttg tgtaacctct 8280

actctccgtt tgcatgatcc gctctgttag atgcattcat tgtagttcgg aagcaagcgt 8340

gtatgaataa agatactgat cactgctgaa aaaaaaaaaa aaaaa 8385

<210>  8
<211>  672
<212>  PRT
<213>  Mus musculus

<400>  8

Met Ala Asp Val Tyr Pro Ala Asn Asp Ser Thr Ala Ser Gln Asp Val
1               5                   10                  15

Ala Asn Arg Phe Ala Arg Lys Gly Ala Leu Arg Gln Lys Asn Val His
            20                  25                  30

Glu Val Lys Asp His Lys Phe Ile Ala Arg Phe Phe Lys Gln Pro Thr
        35                  40                  45

Phe Cys Ser His Cys Thr Asp Phe Ile Trp Gly Phe Gly Lys Gln Gly
    50                  55                  60

Phe Gln Cys Gln Val Cys Cys Phe Val Val His Lys Arg Cys His Glu
65                  70                  75                  80

Phe Val Thr Phe Ser Cys Pro Gly Ala Asp Lys Gly Pro Asp Thr Asp
                85                  90                  95

Asp Pro Arg Ser Lys His Lys Phe Lys Ile His Thr Tyr Gly Ser Pro
            100                 105                 110

Thr Phe Cys Asp His Cys Gly Ser Leu Leu Tyr Gly Leu Ile His Gln
        115                 120                 125

Gly Met Lys Cys Asp Thr Cys Asp Met Asn Val His Lys Gln Cys Val
    130                 135                 140

Ile Asn Val Pro Ser Leu Cys Gly Met Asp His Thr Glu Lys Arg Gly
145                 150                 155                 160

Arg Ile Tyr Leu Lys Ala Glu Val Thr Asp Glu Lys Leu His Val Thr
                165                 170                 175

Val Arg Asp Ala Lys Asn Leu Ile Pro Met Asp Pro Asn Gly Leu Ser
            180                 185                 190

Asp Pro Tyr Val Lys Leu Lys Leu Ile Pro Asp Pro Lys Asn Glu Ser
        195                 200                 205

Lys Gln Lys Thr Lys Thr Ile Arg Ser Thr Leu Asn Pro Gln Trp Asn
    210                 215                 220

Glu Ser Phe Thr Phe Lys Leu Lys Pro Ser Asp Lys Asp Arg Arg Leu
225                 230                 235                 240

Ser Val Glu Ile Trp Asp Trp Asp Arg Thr Thr Arg Asn Asp Phe Met
                245                 250                 255

Gly Ser Leu Ser Phe Gly Val Ser Glu Leu Met Lys Met Pro Ala Ser
            260                 265                 270

Gly Trp Tyr Lys Leu Leu Asn Gln Glu Glu Gly Glu Tyr Tyr Asn Val
        275                 280                 285

Pro Ile Pro Glu Gly Asp Glu Glu Gly Asn Met Glu Leu Arg Gln Lys
    290                 295                 300

Phe Glu Lys Ala Lys Leu Gly Pro Ala Gly Asn Lys Val Ile Ser Pro
305                 310                 315                 320

Ser Glu Asp Arg Lys Gln Pro Ser Asn Asn Leu Asp Arg Val Lys Leu
                325                 330                 335

Thr Asp Phe Asn Phe Leu Met Val Leu Gly Lys Gly Ser Phe Gly Lys
            340                 345                 350

Val Met Leu Ala Asp Arg Lys Gly Thr Glu Glu Leu Tyr Ala Ile Lys
        355                 360                 365

Ile Leu Lys Lys Asp Val Val Ile Gln Asp Asp Asp Val Glu Cys Thr
    370                 375                 380

Met Val Glu Lys Arg Val Leu Ala Leu Leu Asp Lys Pro Pro Phe Leu
385                 390                 395                 400

Thr Gln Leu His Ser Cys Phe Gln Thr Val Asp Arg Leu Tyr Phe Val
                405                 410                 415

Met Glu Tyr Val Asn Gly Gly Asp Leu Met Tyr His Ile Gln Gln Val
            420                 425                 430

Gly Lys Phe Lys Glu Pro Gln Ala Val Phe Tyr Ala Ala Glu Ile Ser
        435                 440                 445

Ile Gly Leu Phe Phe Leu His Lys Arg Gly Ile Ile Tyr Arg Asp Leu
    450                 455                 460

Lys Leu Asp Asn Val Met Leu Asp Ser Glu Gly His Ile Lys Ile Ala
465                 470                 475                 480

Asp Phe Gly Met Cys Lys Glu His Met Met Asp Gly Val Thr Thr Arg
                485                 490                 495

Thr Phe Cys Gly Thr Pro Asp Tyr Ile Ala Pro Glu Ile Ile Ala Tyr
            500                 505                 510

Gln Pro Tyr Gly Lys Ser Val Asp Trp Trp Ala Tyr Gly Val Leu Leu
        515                 520                 525

Tyr Glu Met Leu Ala Gly Gln Pro Pro Phe Asp Gly Glu Asp Glu Asp
    530                 535                 540

Glu Leu Phe Gln Ser Ile Met Glu His Asn Val Ser Tyr Pro Lys Ser
545                 550                 555                 560

Leu Ser Lys Glu Ala Val Ser Ile Cys Lys Gly Leu Met Thr Lys His
                565                 570                 575

Pro Ala Lys Arg Leu Gly Cys Gly Pro Glu Gly Glu Arg Asp Val Arg
            580                 585                 590

Glu His Ala Phe Phe Arg Arg Ile Asp Trp Glu Lys Leu Glu Asn Arg
        595                 600                 605

Glu Ile Gln Pro Pro Phe Lys Pro Lys Val Cys Gly Lys Gly Ala Glu
    610                 615                 620

Asn Phe Asp Lys Phe Phe Thr Arg Gly Gln Pro Val Leu Thr Pro Pro
625                 630                 635                 640

Asp Gln Leu Val Ile Ala Asn Ile Asp Gln Ser Asp Phe Glu Gly Phe
                645                 650                 655

Ser Tyr Val Asn Pro Gln Phe Val His Pro Ile Leu Gln Ser Ala Val
            660                 665                 670

<210>  9
<211>  3524
<212>  DNA
<213>  Mus musculus

<400>  9
gccgcgagta ggaagcgcga gcgccgggcg cgggctgtca gtgagcgtgg ggccagccag      60

cgagccagag agccggagag agccagagag agcggctcgg ctcccagctc cgagtagcgc     120

agccgcccgg ctctccccgg caccgccgcc accaccgcac ctcatcagca ccgccacctc     180

gccgccgccc ccgcccaccc cggccctccc cggctgctgc tccccggcgg aggcaagagg     240

tggttggggg gaaccatggc tgacgtttac ccggccaacg actccacggc gtctcaggac 300

gtggccaacc gcttcgcccg caaaggggcg ctgaggcaga agaacgtgca tgaggtgaaa 360

gaccacaaat tcatcgcccg cttcttcaag caacccacct tctgcagcca ctgcaccgac 420

ttcatctggg ggtttgggaa acaaggcttc cagtgccaag tttgctgttt tgtggttcat 480

aagaggtgcc atgagttcgt tacgttctct tgtccgggtg cggataaggg acctgacact 540

gacgacccca ggagcaagca caagttcaaa atccacacat acggaagccc taccttctgt 600

gatcactgtg ggtccctgct ctatggactt atccaccaag ggatgaaatg tgacacctgc 660

gacatgaatg ttcacaacca gtgtgtgatc aatgacccta gcctctgcgg aatggatcac 720

acagagaaga gggggcggat ttatctgaag gctgaggtca ctgatgaaaa gctccacgtc 780

acggtacgag atgcaaaaaa tctaatccct atggatccaa atgggctttc ggatccttat 840

gtgaagctga aactaatccc tgaccccaag aatgagagca aacagaaaac caaaaccatc 900

cgctccaacc tgaatcctca gtggaatgag tccttcacgt tcaaattaaa accttcagac 960

aaagaccggc gactgtctgt agaaatctgg gactgggatc ggacgactcg gaatgacttc 1020

atgggatccc tttcctttgg tgtctcagag ctaatgaaga tgccggccag tggatggtat 1080

aaagctcaca accaagaaga gggcgaatat tacaacgtgc ccattccaga aggagatgaa 1140

gaaggcaaca tggaactcag gcagaagttt gagaaagcca agctaggtcc tgttggtaac 1200

aaagtcatca gcccttcaga agacagaaag caaccatcca acaacctgga cagagtgaaa 1260

ctcacagact tcaacttcct catggtgctg gggaagggga gttttgggaa ggtgatgctt 1320

gctgacagga agggaacgga ggaactgtac gccatcaaga tcctgaagaa ggacgtggtg 1380

atccaggacg acgacgtgga gtgcaccatg gtggagaagc gcgtgctggc cctgctggac 1440

aagccgccat ttctgacaca gctgcactcc tgcttccaga cagtggaccg gctgtacttc 1500

gtcatggaat acgtcaacgg cggggatctt atgtaccaca ttcagcaagt cgggaaattt 1560

aaggagccac aagcagtatt ctacgcagcc gagatctcca tcggactgtt cttccttcat 1620

aaaagaggga tcatttacag ggatctgaag ctgaacaatg tcatgctgaa ctcagaaggg 1680

cacatcaaaa tcgccgactt cgggatgtgc aaggaacaca tgatggatgg agtcacgacc 1740

aggaccttct gcggaactcc ggactacatt gccccagaga taatcgctta ccagccgtac 1800

gggaagtctg tagattggtg ggcgtacggt gtgctgctgt acgagatgct agccgggcag 1860

cctccgtttg atggtgaaga tgaagatgaa ctgtttcagt ctataatgga gcacaacgtg 1920

tcctacccca aatccttgtc caaggaagcc gtctccatct gcaaaggact tatgaccaaa 1980

cagcctgcca agcgactggg ctgcgggccc gagggagaga gggatgtcag agagcatgcc 2040

ttcttcagga ggatcgactg ggagaaactg gagaacaggg agatccaacc accattcaag 2100

cccaaagtgt gtggcaaagg agcagaaaac tttgacaagt tcttcacgcg aggacagcct 2160

gtcttaacac caccagatca gctggtcatt gctaacatag accaatctga ttttgaaggg 2220

ttctcgtatg tcaaccccca gtttgtgcac ccaatcttgc aaagtgcagt atgaactcaa 2280

aaatgaaaga tcaaatgcct ccccagcccc cgcatcccca tcagtgggaa gtgatcctta 2340

accataaaaa tttaaggcta tggctttgta ttttgttcca cacagaggcc tggaaatttt 2400

agggacatta gtaatacatt atcaactttt ttcctcaccc cccaaccaaa agcattatct 2460

tagtggatga tgacataaag tacagagtat agtttaatta tgtagaagtc atgtccggct 2520

acaagttgat tcttcctaaa aatcaaagag acttggaccc tattttttgg tacgatttga 2580

tatattctcc atactttcca tttcttggat tttcagtatc agtatcaacc agagatagta 2640

aagtgaccca ccctaactca aggaacactt ccacccaaga tgtctttgga acaaagacag 2700

gaagtccaaa gagaaagcaa agagaggttg ggggctcata tattcaagac cacatagctt 2760

ctattcttcg tttcaatgga aacatcccta gactcagaga gggcaggatg aacctgatca 2820

ctgttcccag tcatcatagc acaaccatag tgcacagttt aagaatgaaa gaaaacttca 2880

gacaaatatt cactgaatct accatctgta atcccctgct tagttgataa caatctctat 2940

aactcattct ttttaagagg ccaaatcatc taagggcttt gctaaacaag catatgaaat 3000

catttcagat caaggataaa ccatgtgtac gttcattcta atctctggga gatgactctt 3060

caatccaggg tgccatcagt aatcatgcca ctgttcacga gtgttgttag ccaaccccac 3120

acacacaata ttttgctacc tttgtaggta cccttcctaa gaagatgaaa tgtatgaccc 3180

catccccccc ttttttttta ctatttattt aataagctgc agtgtcgttt atggaagtac 3240

aatgtatagt aatttagtca aagtattgac tagcctcagt ccctctagat tgattttcct 3300

cctttctctc gccccacatc cacttagaga tgaaaaagag catagtttgg gttcccattc 3360

tcagttccta ttgaacggca catgtggaac tgtaggatca agatacttta tgctgacatc 3420

aactctttga tgttgtcttg ctagaaggat tttaatatgt tttgcgagtg catcatgcaa 3480

tggattttgc atgtttataa taaaccttaa taacgagtta agtg 3524

<210> 10
<211> 672
<212> PRT
<213> Mus musculus

<400> 10

Met Ala Asp Val Tyr Pro Ala Asn Asp Ser Thr Ala Ser Gln Asp Val
1               5                   10                  15

Ala Asn Arg Phe Ala Arg Lys Gly Ala Leu Arg Gln Lys Asn Val His
            20                  25                  30

Glu Val Lys Asp His Lys Phe Ile Ala Arg Phe Phe Lys Gln Pro Thr
        35                  40                  45

Phe Cys Ser His Cys Thr Asp Phe Ile Trp Gly Phe Gly Lys Gln Gly
    50                  55                  60

Phe Gln Cys Gln Val Cys Cys Phe Val Val His Lys Arg Cys His Glu
65                  70                  75                  80

Phe Val Thr Phe Ser Cys Pro Gly Ala Asp Lys Gly Pro Asp Thr Asp
                85                  90                  95

Asp Pro Arg Ser Lys His Lys Phe Lys Ile His Thr Tyr Gly Ser Pro
            100                 105                 110

Thr Phe Cys Asp His Cys Gly Ser Leu Leu Tyr Gly Leu Ile His Gln
        115                 120                 125

Gly Met Lys Cys Asp Thr Cys Asp Met Asn Val His Asn Gln Cys Val
    130                 135                 140

Ile Asn Asp Pro Ser Leu Cys Gly Met Asp His Thr Glu Lys Arg Gly
145                 150                 155                 160

Arg Ile Tyr Leu Lys Ala Glu Val Thr Asp Glu Lys Leu His Val Thr
                165                 170                 175

Val Arg Asp Ala Lys Asn Leu Ile Pro Met Asp Pro Asn Gly Leu Ser
            180                 185                 190

Asp Pro Tyr Val Lys Leu Lys Leu Ile Pro Asp Pro Lys Asn Glu Ser
        195                 200                 205

Lys Gln Lys Thr Lys Thr Ile Arg Ser Asn Leu Asn Pro Gln Trp Asn
    210                 215                 220

Glu Ser Phe Thr Phe Lys Leu Lys Pro Ser Asp Lys Asp Arg Arg Leu
225                 230                 235                 240

Ser Val Glu Ile Trp Asp Trp Asp Arg Thr Thr Arg Asn Asp Phe Met
                245                 250                 255

Gly Ser Leu Ser Phe Gly Val Ser Glu Leu Met Lys Met Pro Ala Ser
            260                 265                 270

Gly Trp Tyr Lys Ala His Asn Gln Glu Glu Gly Glu Tyr Tyr Asn Val
        275                 280                 285

Pro Ile Pro Glu Gly Asp Glu Glu Gly Asn Met Glu Leu Arg Gln Lys
    290                 295                 300

Phe Glu Lys Ala Lys Leu Gly Pro Val Gly Asn Lys Val Ile Ser Pro
305                 310                 315                 320

Ser Glu Asp Arg Lys Gln Pro Ser Asn Asn Leu Asp Arg Val Lys Leu
                325                 330                 335

Thr Asp Phe Asn Phe Leu Met Val Leu Gly Lys Gly Ser Phe Gly Lys
            340                 345                 350

Val Met Leu Ala Asp Arg Lys Gly Thr Glu Glu Leu Tyr Ala Ile Lys
        355                 360                 365

Ile Leu Lys Lys Asp Val Val Ile Gln Asp Asp Asp Val Glu Cys Thr
    370                 375                 380

Met Val Glu Lys Arg Val Leu Ala Leu Leu Asp Lys Pro Pro Phe Leu
385                 390                 395                 400

Thr Gln Leu His Ser Cys Phe Gln Thr Val Asp Arg Leu Tyr Phe Val
                405                 410                 415

Met Glu Tyr Val Asn Gly Gly Asp Leu Met Tyr His Ile Gln Gln Val
            420                 425                 430

Gly Lys Phe Lys Glu Pro Gln Ala Val Phe Tyr Ala Ala Glu Ile Ser
        435                 440                 445

Ile Gly Leu Phe Phe Leu His Lys Arg Gly Ile Ile Tyr Arg Asp Leu
    450                 455                 460

Lys Leu Asn Asn Val Met Leu Asn Ser Glu Gly His Ile Lys Ile Ala
465                 470                 475                 480

Asp Phe Gly Met Cys Lys Glu His Met Met Asp Gly Val Thr Thr Arg
                485                 490                 495

Thr Phe Cys Gly Thr Pro Asp Tyr Ile Ala Pro Glu Ile Ile Ala Tyr
            500                 505                 510

Gln Pro Tyr Gly Lys Ser Val Asp Trp Trp Ala Tyr Gly Val Leu Leu
        515                 520                 525

Tyr Glu Met Leu Ala Gly Gln Pro Pro Phe Asp Gly Glu Asp Glu Asp

530                 535                 540

Glu Leu Phe Gln Ser Ile Met Glu His Asn Val Ser Tyr Pro Lys Ser
545                 550                 555                 560

Leu Ser Lys Glu Ala Val Ser Ile Cys Lys Gly Leu Met Thr Lys Gln
                565                 570                 575

Pro Ala Lys Arg Leu Gly Cys Gly Pro Glu Gly Glu Arg Asp Val Arg
            580                 585                 590

Glu His Ala Phe Phe Arg Arg Ile Asp Trp Glu Lys Leu Glu Asn Arg
        595                 600                 605

Glu Ile Gln Pro Pro Phe Lys Pro Lys Val Cys Gly Lys Gly Ala Glu
    610                 615                 620

Asn Phe Asp Lys Phe Phe Thr Arg Gly Gln Pro Val Leu Thr Pro Pro
625                 630                 635                 640

Asp Gln Leu Val Ile Ala Asn Ile Asp Gln Ser Asp Phe Glu Gly Phe
                645                 650                 655

Ser Tyr Val Asn Pro Gln Phe Val His Pro Ile Leu Gln Ser Ala Val
            660                 665                 670

<210>  11
<211>  3702
<212>  DNA
<213>  Rattus norvegicus

<400>  11
gccgcgagta ggaagcgcga gcgccaggcg ccgggctgtc agtgagcgtg gggccagcca      60

gagagcgaga gagccggaga gagccagaga gagccagaga gagcggctca gctcccagct     120

ccaagcagcg cagcgcccgc ccggctctcc ccggccaccg ccgccaccac cgcacctcag     180

caccgccacc tcggccgccg cccccgccca ccccggccct ccccggctgc tgctccccgg     240

cggaggcaag aggtggttgg gggggaccat ggctgacgtt tacccggcca acgactccac     300

ggcgtctcag gacgtggcca accgcttcgc ccgcaaaggg gcgctgaggc agaagaacgt     360

gcatgaggtg aaagaccaca aattcatcgc ccgcttcttc aagcaaccca ccttctgcag     420

ccactgcacc gacttcatct gggggtttgg aaaacaaggc ttccagtgcc aagtttgctg     480

ttttgtggtt cacaagaggt gccatgagtt tgttactttc tcttgtccgg gtgcggataa     540

gggacctgac actgatgacc ccagaagcaa gcacaagttc aaaatccaca cctatggaag     600

ccctaccttc tgtgatcact gtgggtccct gctctacgga cttatccacc aagggatgaa     660

atgcgacacc tgcgacatga atgttcacaa gcagtgcgtg atcaatgtcc ccagcctctg 720

cggaatggat cacacagaga agagggggcg gatttacctg aaggcagagg tcacagatga 780

aaagctgcac gtcaccgtac gagatgcaaa aaatctaatc cctatggatc caaatgggct 840

ttcggatcct tacgtgaagc tgaaacttat tcctgacccc aagaatgaga gcaaacagaa 900

aaccaaaacc atccgatcca cactgaaccc tcagtggaat gagtccttca cgttcaaatt 960

aaaaccttca gacaaagacc ggcgactgtc cgtagaaatc tgggactggg atcggacgac 1020

acggaatgac ttcatgggct ccctttcctt cggcgtctca gagctgatga agatgccagc 1080

cagtggatgg tacaagttgc tcaaccaaga ggagggtgaa tactacaatg tgcccattcc 1140

agaaggagat gaagaaggca acgtggaact caggcagaag ttcgagaaag ccaagctggg 1200

ccccgctgga aacaaagtca tcagcccttc agaagacagg aagcagccat ctaacaacct 1260

ggacagggtg aaactcacag acttcaactt cctcatggtg ctggggaagg ggagttttgg 1320

aaaggtgatg cttgctgaca ggaagggaac agaggaactg tacgccatca aaatcctgaa 1380

gaaggacgtg gtgatccagg atgacgacgt ggagtgcacc atggtggaga agcgggttct 1440

ggccctgctc gacaagcccc cgttcctgac acagctgcac tcctgcttcc agacagtgga 1500

ccggctgtac ttcgtcatgg aatacgtcaa cggtggggac ctcatgtacc acattcagca 1560

agtcggaaaa tttaaggagc cacaagcagt attctatgca gccgagatct ccatcggact 1620

gttctttctt cacaaaagag gaatcattta cagggatctg aagctggaca acgtcatgct 1680

ggactcagaa gggcatatca aaatcgccga cttcgggatg tgcaaggaac acatgatgga 1740

cggggtcacg accaggacct tctgtgggac tccggattac attgccccag agataatcgc 1800

ttaccagcca tatggaaagt ctgtggactg gtgggcgtac ggcgtgctcc tgtatgagat 1860

gctagctggg cagcttatga ccaaacaccc tgccaagcgg ctgggctgcg ggcccgaggg 1920

ggaaagggat gtcagagagc atgccttctt taggaggatc gactgggaga agttggagaa 1980

cagggagatc caaccgccat tcaagcccaa agtgtgcggc aaaggagcag aaaactttga 2040

caagttcttc acacgaggcc agcctgtctt aacaccacca gatcagctgg tcatcgctaa 2100

catagaccag tctgattttg aagggttctc gtatgtcaac ccccagtttg tgcacccaat 2160

cttgcaaagt gcagtatgaa actcagaaac aaaagatcta atgcctccct agcccccaat 2220

ctccccagca gtgggaagtg attcttaacc ataaaatttt aaggctatag ccttgtattt 2280

tgttccacac agaggcctga aaattctggg gatattagtc cataagtgat caactttctt 2340

cccccaccca atcccaaacc aaaaaacatt atcttagtgg atgatgacat aatatacaga 2400

gtatagttta attatgtaga agtcacatct ggcttcaagt taattctttc taggaaacaa 2460

agagacttgg accctatttt ttggtacgat ttaatatatt ctccatacct ttcatatttt 2520

ggattttcac tatccaaatc aaccagagat aataaagtga acccacctga actcaaggga 2580

tggaaacatt tctgcccaag atatctttgg aattaaagaa caggaagccc aaacagaaaa 2640

caaagagagg cagagtctca tatattcaag acctcgttgc ttctattttc tgcttcaatg 2700

gaaacagtcc ctagagtctg agagggcagg atgaacctga tcactgttcc caatcatcat 2760

agcacaacca tagtgcatag tttgaaaatg aaagaaaact tcagacagat gttcgttgaa 2820

tctatcatat gtactcccct gctcggttga taactatctc gataactcat tctttttaag 2880

aggccaaaat catctaagga ctttgctaaa caaacatgtg aaatcatttc agatcaagga 2940

taaaccatgt gtatgttcat tttaatctct gggagatgac tcttcaatcc agggtgccat 3000

cagtaatcat gccactgttc acgagtgttg ttagccaacc ccccgccaca taataatatt 3060

ttgctacctt tgtgggtacc cttcctagga agctaaaatg tatgccccat ccccttttgt 3120

actatttatt taataagccg cagtgtcgtt tatgaaagta caatgtatag taacttaatc 3180

aaagtactga ctagcatcag tccctatagg ttgattttcc tcctttctct agccccacat 3240

ccacttagag atgagaaaaa aaagagtata ttttgggttc caattcccag ttctaattga 3300

atggcacaca tggagctgta ggatcaagac actttatgct tatatcaact caaagatgtt 3360

ttcttgctag aaggatttta atatgttttg cgagtgcatc atgcaatgga ttttgcatgt 3420

ttataataaa ccttaataac aagttaatct atattattga tataattgta tcaagtataa 3480

agagtattat gataatttta taagacacaa ttgtgctata tttgtgatgc tcatgtttcc 3540

agtctgcttt aagattaagt gttagcttta ttcattgctg ctggggcctc tattacccgc 3600

taccccacac actggcactc tatcagatat attaattttt taatgtagat gtaattttaa 3660

aatgaatggc tactttacac gtttaactag gcttttacta ta 3702

<210> 12
<211> 3785
<212> DNA
<213> Rattus norvegicus

<400> 12
atgacgatct gtatcaagtg tgagttttgc tgttttgtgg ttcacaagag gtgccatgag 60

tttgttactt tctcttgtcc gggtgcggat aagggacctg acactgatga ccccagaagc 120

aagcacaagt tcaaaatcca cacctatgga agccctacct tctgtgatca ctgtgggtcc 180

ctgctctacg gacttatcca ccaagggatg aaatgcgaca cctgcgacat gaatgttcac 240

aagcagtgcg tgatcaatgt ccccagcctc tgcggaatgg atcacacaga gaagaggggg 300

cggatttacc tgaaggcaga ggtcacagat gaaaagctgc acgtcaccgt acgagatgca 360

aaaaatctaa tccctatgga tccaaatggg ctttcggatc cttacgtgaa gctgaaactt 420

attcctgacc ccaagaatga gagcaaacag aaaaccaaaa ccatccgatc cacactgaac 480

cctcagtgga atgagtcctt cacgttcaaa ttaaaacctt cagacaaaga ccggcgactg 540

tccgtagaaa tctgggactg ggatcggacg acacggaatg acttcatggg ctccctttcc 600

ttcggcgtct cagagctgat gaagatgcca gccagtggat ggtacaagtt gctcaaccaa 660

gaggagggtg aatactacaa tgtgcccatt ccagaaggag atgaagaagg caacgtggaa 720

ctcaggcaga agttcgagaa agccaagctg ggccccgctg gaaacaaagt catcagccct 780

tcagaagaca ggaagcagcc atctaacaac ctggacaggg tgaaactcac agacttcaac 840

ttcctcatgg tgctggggaa ggggagtttt ggaaaggtga tgcttgctga caggaaggga 900

acagaggaac tgtacgccat caaaatcctg aagaaggacg tggtgatcca ggatgacgac 960

gtggagtgca ccatggtgga gaagcgggtt ctggccctgc tcgacaagcc cccgttcctg 1020

acacagctgc actcctgctt ccagacagtg gaccggctgt acttcgtcat ggaatacgtc 1080

aacggtgggg acctcatgta ccacattcag caagtcggaa aatttaagga gccacaagca 1140

gtattctatg cagccgagat ctccatcgga ctgttctttc ttcacaaaag aggaatcatt 1200

tacagggatc tgaagctgga caacgtcatg ctggactcag aagggcatat caaaatcgcc 1260

gacttcggga tgtgcaagga acacatgatg gacggggtca cgaccaggac cttctgtggg 1320

actccggatt acattgcccc agagataatc gcttaccagc catatggaaa gtctgtggac 1380

tggtgggcgt acggcgtgct cctgtatgag atgctagctg ggcagcctcc gttcgatggc 1440

gaagacgaag atgaactgtt tcagtctata atggagcaca atgtgtccta ccccaaatcc 1500

ttgtccaagg aagctgtctc catctgcaaa gggcttatga ccaaacaccc tgccaagcgg 1560

ctgggctgcg ggcccgaggg ggaaagggat gtcagagagc atgccttctt taggaggatc 1620

gactgggaga agttggagaa cagggagatc caaccgccat tcaagcccaa agtgtgcggc 1680

aaaggagcag aaaactttga caagttcttc acacgaggcc agcctgtctt aacaccacca 1740

gatcagctgg tcatcgctaa catagaccag tctgattttg aagggttctc gtatgtcaac 1800

ccccagtttg tgcacccaat cttgcaaagt gcagtatgaa actcagaaac aaaagatcta 1860

atgcctccct agcccccaat ctccccagca gtgggaagtg attcttaacc ataaaatttt 1920

aaggctatag ccttgtattt tgttccacac agaggcctga aaattctggg gatattagtc 1980

cataagtgat caactttctt cccccaccca atcccaaacc aaaaaacatt atcttagtgg 2040

atgatgacat aatatacaga gtatagttta attatgtaga agtcacatct ggcttcaagt 2100

taattctttc taggaaacaa agagacttgg accctatttt ttggtacgat ttaatatatt 2160

ctccatacct ttcatatttt ggattttcac tatccaaatc aaccagagat aataaagtga 2220

acccacctga actcaaggga tggaaacatt tctgcccaag atatctttgg aattaaagaa 2280

caggaagccc aaacagaaaa caaagagagg cagagtctca tatattcaag acctcgttgc 2340

ttctattttc tgcttcaatg gaaacagtcc ctagagtctg agagggcagg atgaacctga 2400

tcactgttcc caatcatcat agcacaacca tagtgcatag tttgaaaatg aaagaaaact 2460

tcagacagat gttcgttgaa tctatcatat gtactcccct gctcggttga taactatctc 2520

gataactcat tctttttaag aggccaaaat catctaagga ctttgctaaa caaacatgtg 2580

aaatcatttc agatcaagga taaaccatgt gtatgttcat tttaatctct gggagatgac 2640

tcttcaatcc agggtgccat cagtaatcat gccactgttc acgagtgttg ttagccaacc 2700

ccccgccaca taataatatt ttgctacctt tgtgggtacc cttcctagga agctaaaatg 2760

tatgccccat ccccttttgt actatttatt taataagccg cagtgtcgtt tatgaaagta 2820

caatgtatag taacttaatc aaagtactga ctagcatcag tccctatagg ttgattttcc 2880

tcctttctct agccccacat ccacttagag atgagaaaaa aaagagtata ttttgggttc 2940

caattcccag ttctaattga atggcacaca tggagctgta ggatcaagac actttatgct 3000

tatatcaact caaagatgtt ttcttgctag aaggatttta atatgttttg cgagtgcatc 3060

atgcaatgga ttttgcatgt ttataataaa ccttaataac aagttaatct atattattga 3120

tataattgta tcaagtataa agagtattat gataatttta taagacacaa ttgtgctata 3180

tttgtgatgc tcatgtttcc agtctgcttt aagattaagt gttagcttta ttcattgctg 3240

ctggggcctc tattacccgc taccccacac actggcactc tatcagatat attaattttt 3300

taatgtagat gtaattttaa aatgaatggc tactttacac gtttaactag gcttttacta 3360

tatatgtgta catatgtata tatatatatt tgattctacc tgcaaacccc ggttttgttt 3420

gtggggatta tttttgagat gagactctgt taacttcttc atgttatcct ccgttcaggt 3480

tcctctacca tccctcccct tggggacagc tggaaatgtt taattccagt attcctgata 3540

tgttcatata tatgtggcat tgttacagca cctgggctga attccgtgtg tgtgtgtgtg 3600

tgtgtgtgtg tgtgtgtgtg tgtgtgtgtg tgtgtgtctg tgtgtctgtc tgtctgtttc 3660

tgtctgtttc tgtctgtttc tgtctgtctg tctgattctt ttcagcctat gtctttgggg 3720

tctctactga tgaaagggtg ggaggattcc tttctgagtg tgcatatgga cacaacaaaa 3780

atatg 3785

## Description

### Technical Field

The present invention relates to a method for diagnosing cancer, a cancer diagnosis composition, a cancer diagnosis kit, a method for evaluating a state of cancer, and a method for screening agents for preventing and/or treating cancer.

### Background Art

A living cell contains a huge number of intracellular signaling pathways for controlling or adjusting gene expression in response to extracellular signals. Phosphorylation caused by protein kinase activates a target protein in such an intracellular signaling pathway, and plays a significant role in cell growth (see Non Patent Literatures 1 to 3). Protein kinase C (PKC), that is, one of phospholipid-dependent serine/threonine kinases, is one of the most significant kinases, and is classified into three subfamilies based on the structure and features of activation. Specifically, the subfamilies are conventional or classical PKC (cPKC; α, βI, βII, and γ), novel or non-classical PKC (nPKC; δ, ε, η, and θ), and atypical PKC (ζ, ι, and λ) (see Non Patent Literatures 4 to 6).

Among these PKC isozymes, it is known that PKCα exhibits very slight activity in a normal tissue, and is overexpressed or highly activated in most of cancer cells (for example, liver cancer, breast cancer, and melanoma) (see Non Patent Literatures 7 to 10).

### Citation List

### Non Patent Literature

Non Patent Literature 1: Adjei, A. A., and Hidalgo, M. (2005) Intracellular signal transduction pathway proteins as targets for cancer therapy. J. Clin. Oncol. 23, 5386-5403.
Non Patent Literature 2: Cross, T. G., Scheel-Toellner, D., Henriquez, N. V, Deacon, E., Salmon, M., and Lord, J. M. (2000) Serine/threonine protein kinases and apoptosis. Exp. Cell Res. 256, 34-41.
Non Patent Literature 3: Bode, A. M., and Dong, Z. (2005) Signal transduction pathways in cancer development and as targets for cancer prevention. Prog. Nucleic Acid Res. Mol. Biol. 79, 237-297.
Non Patent Literature 4: Newton, A. C. (1997) Regulation of protein kinase C. Curr. Opin. Cell Biol. 9, 161-167.
Non Patent Literature 5: Blobe, G. C., Obeid, L. M., and Hannun, Y. A. (1994) Regulation of protein kinase C and role in cancer biology. Cancer Metastasis Rev. 13,411-431.
Non Patent Literature 6: Webb, B. L. J., Hirst, S. J., and Giembycz, M. A. (2000) Protein kinase C isoenzymes: a review of their structure, regulation and role in regulating airways smooth muscle tone and mitogenesis. Br. J. Pharmacol. 130, 1433-1452.
Non Patent Literature 7: Oka, M., and Kikkawa, U. (2005) Protein kinase C in melanoma. Cancer Metast. Rev. 24, 287-300.
Non Patent Literature 8: Mackay, H. J., and Twelves, C. J. (2003) Protein kinase C: a target for anticancer drugs? Endocrin. Relat. Cancer 10, 389-396.
Non Patent Literature 9: Hofmann, J. (2004) Protein kinase C isozymes as potential targets for anticancer therapy. Curr. Cancer Drug Targets 4, 125-146.
Non Patent Literature 10: Goekjian, P. G., and Jirousek, M. R. (2001) Protein kinase C inhibitors as novel anticancer drugs. Expert. Opin. Investig. Drugs 10, 2117-2140.
Non Patent Literature 11: O'Brian, C. A., Chu, F., Bornmann, W. G., and Maxwell, D. S. (2006) Protein kinase Cα and ε small-molecule targeted therapeutics: a new roadmap to two holy grails in drug discovery? Expert. Rev. Anticancer Ther. 6, 175-186.

### Summary of Invention

### Technical Problem

An object of the present invention is to provide a novel biomarker for cancer.

### Solution to Problem

PKCα has been reported to be overexpressed or highly activated in most of cancer cells as described above, but is presumed not to be excreted in urine due to the functions of the glomerulus of the renal corpuscle. This is because PKCα is a negatively charged molecule having a molecular weight of 82 kDa, and the glomerulus of the renal corpuscle has two functions, that is, a size barrier effect of not allowing a protein molecule having a molecular weight of 60 kDa or more to pass therethrough, and a charge barrier through which a negatively charged substance cannot pass because electrically repulsed.

The present inventors found that active protein kinase Cα (active PKCα) can be detected in urine of a cancer patient contrary to the presumption, resulting in accomplishing the present invention.

Specifically, the present invention encompasses, for example, the following [1] to [9]:
[1] A method for diagnosing cancer comprising: a step of detecting active protein kinase Cα (active PKCα) in urine.
[2] The method according to [1], wherein the cancer includes urinary cancer.
[3] The method according to [1] or [2], wherein the cancer includes urothelial carcinoma.
[4] The method according to any one of [1] to [3], wherein the cancer includes non-muscle-invasive cancer.
[5] A cancer diagnosis composition comprising: an antibody or a substrate peptide for detecting active PKCα, wherein diagnosis is made using urine of an examinee.
[6] A cancer diagnosis kit comprising: an antibody or a substrate peptide for detecting active PKCα, wherein diagnosis is made using urine of an examinee.
[7] A method for evaluating a state of cancer, wherein active PKCais detected by reacting an antibody to the active PKCawith urine of an examinee, and a state of the cancer is evaluated with an obtained detection result used as an index.
[8] A method for evaluating a state of cancer by using, as an index, a detection result obtained by reacting a substrate peptide of active PKCawith urine of an examinee, and detecting the peptide having been phosphorylated.
[9] A method for screening agents for preventing and/or treating cancer comprising: a step of administering a candidate substance to a urinary cancer model non-human mammal; a step of detecting active PKCα in urine of the non-human mammal; and a step of selecting an intended agent for preventing and/or treating cancer with an obtained result used as an index.

### Advantageous Effects of Invention

According to the present invention, a method for diagnosing cancer, a cancer diagnosis composition, a cancer diagnosis kit, a method for evaluating a state of cancer, and a method for screening agents for preventing and/or treating cancer, in which active PKCα in urine is used as a biomarker, can be provided. The present invention uses a biomarker present in urine, and hence is simple and non-invasive. As described below, specificity against and sensitivity to early stage cancer are also high.

### Brief Description of Drawings

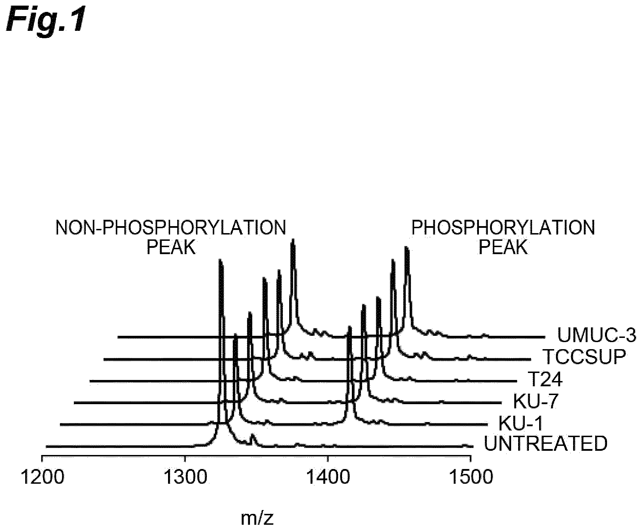

[Figure 1] Figure 1 is a diagram illustrating MALDI-TOF MS spectra obtained as a result of analysis by MALDI-TOF MS of a phosphorylation reaction of a substrate peptide in bladder cancer-derived cultured cells (KU-1, KU-7, T24, TCCSUP, and UMUC-3).

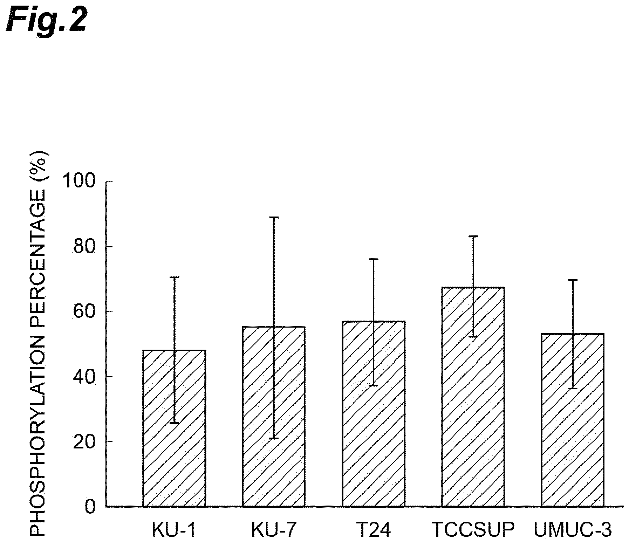

[Figure 2] Figure 2 is a graph illustrating a phosphorylation percentage (%) of the substrate peptide in the bladder cancer-derived cultured cells (KU-1, KU-7, T24, TCCSUP, and UMUC-3).

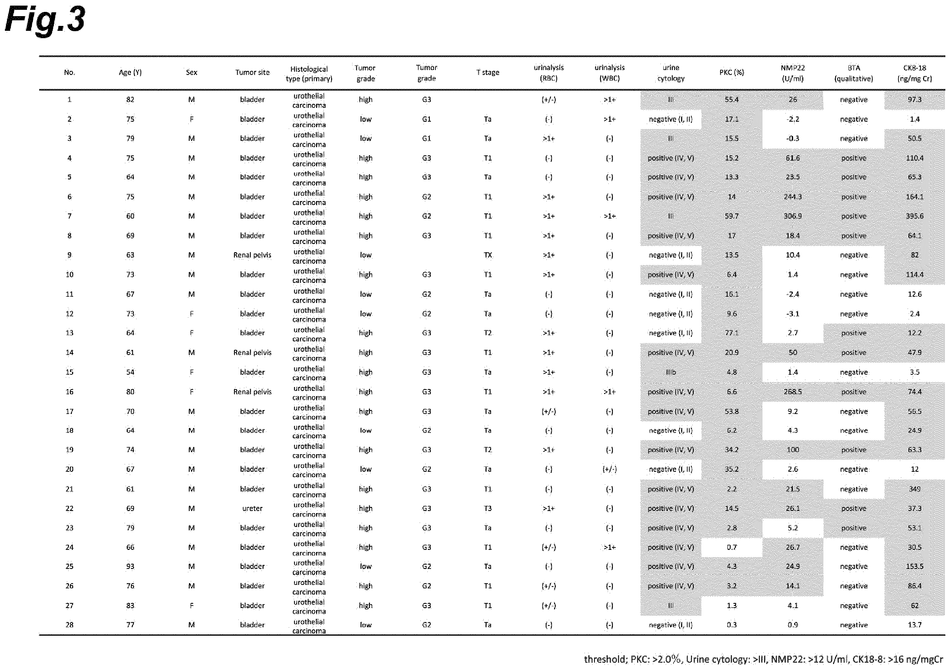

[Figure 3] Figure 3 illustrates case information on urothelial carcinoma patients. Each gray cell indicates that a corresponding result is positive or a corresponding numerical value is equal to or larger than a threshold. "Histological type (primary)" indicates a histological type (primary) of cancer, "urinalysis (RBC)" indicates urinalysis (erythrocyte), "urinalysis (WBC)" indicates urinalysis (leukocyte), and "urine cytology" indicates urine cytology.

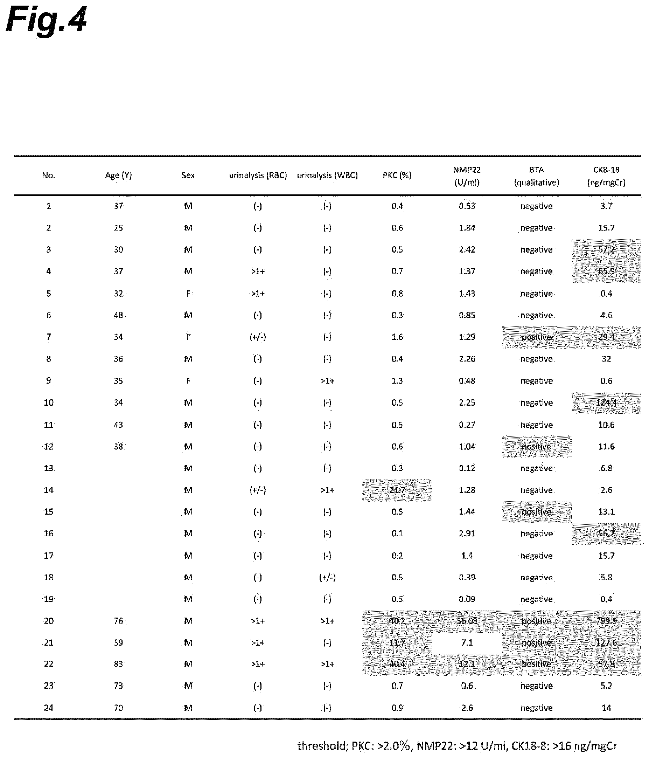

[Figure 4] Figure 4 illustrates case information on non-urothelial carcinoma patients. Each gray cell indicates that a corresponding result is positive or a corresponding numerical value is equal to or larger than a threshold. "Urinalysis (RBC)" indicates urinalysis (erythrocyte), and "urinalysis (WBC)" indicates urinalysis (leukocyte).

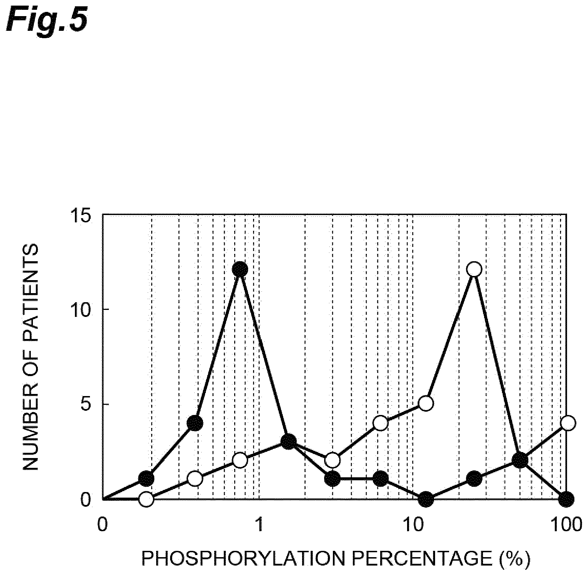

[Figure 5] Figure 5 is a histogram illustrating phosphorylation percentages of a substrate peptide in urine samples obtained from urothelial carcinoma patients and non-urothelial carcinoma patients. A line with white circles indicate data of the urothelial carcinoma patients, and a line with black circles indicate data of the non-urothelial carcinoma patients.

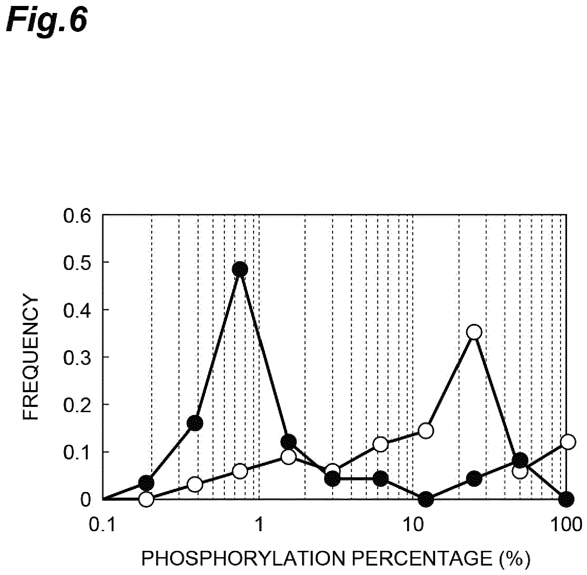

[Figure 6] Figure 6 is a graph illustrating, in the form of a probability density function, the relationship between the phosphorylation percentages and frequencies of a substrate peptide in urine samples obtained from urothelial carcinoma patients and non-urothelial carcinoma patients. A line with white circles indicates data of the urothelial carcinoma patients, and a line with black circles indicates data of the non-urothelial carcinoma patients.

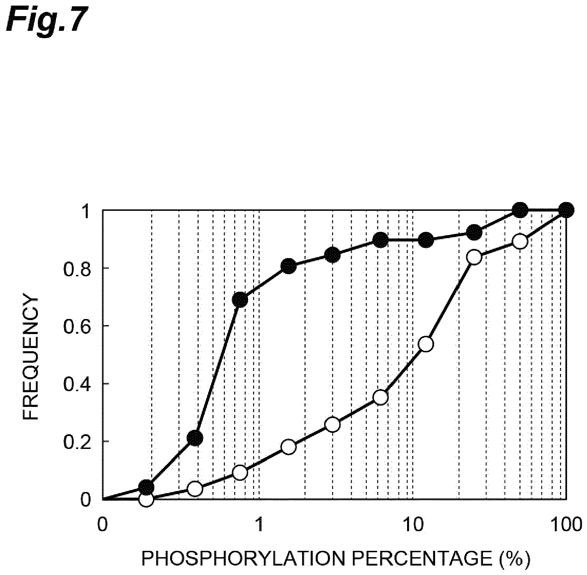

[Figure 7] Figure 7 is a graph illustrating, in the form of a cumulative distribution function, the relationship between the phosphorylation percentages and frequencies of a substrate peptide in urine samples obtained from urothelial carcinoma patients and non-urothelial carcinoma patients. A line with white circles indicates data of the urothelial carcinoma patients, and a line with black circles indicates data of the non-urothelial carcinoma patients.

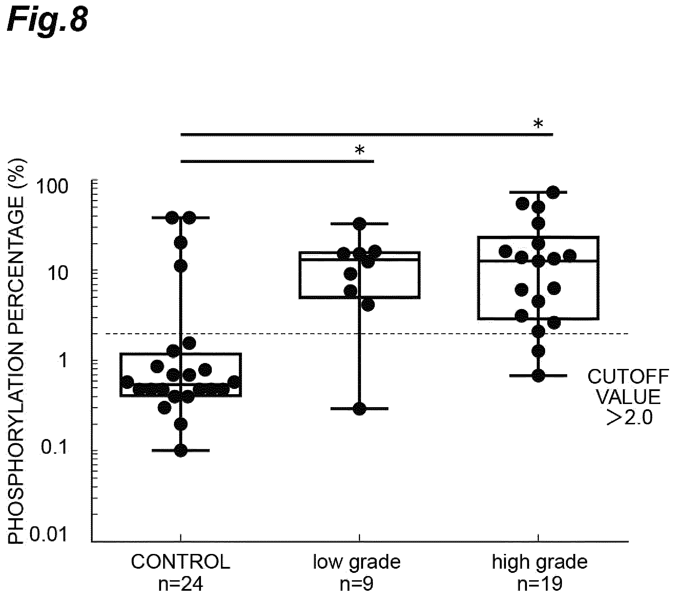

[Figure 8] Figure 8 is a graph illustrating the phosphorylation percentages of a substrate peptide in urine samples obtained from low grade urothelial carcinoma patients and high grade urothelial carcinoma patients (^{∗}: P < 0.05). A dotted line indicates a cutoff value (phosphorylation percentage of 2%).

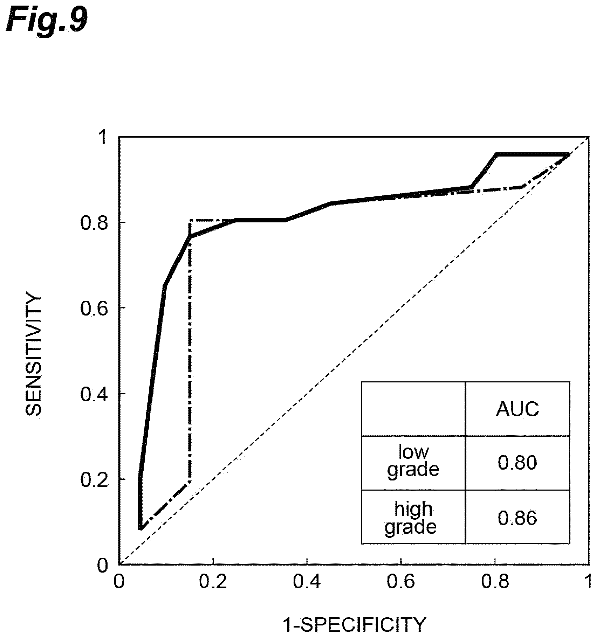

[Figure 9] Figure 9 illustrates receiver operating characteristic (ROC) curves of diagnosis based on the phosphorylation percentages of a substrate peptide using urine samples. A dashed line indicates an ROC curve of low grade urothelial carcinoma patients, and a solid line indicates an ROC curve of high grade urothelial carcinoma patients.

### Description of Embodiments

In the present invention, based on finding that active PKCα is present in urine of a cancer patient, the active PKCα present in urine is used as a cancer marker.

### [Active PKCα]

Active PKCα means PKCα activated by phosphorylating the 497th threonine residue, the 638th threonine residue, and the 657th serine residue in human PKCα, or corresponding threonine residues and serine residue in PKCα of another animal.

### [Target Cancer]

Cancer (type of cancer) to be diagnosed or evaluated is not especially limited, and two or more types of cancer can be targets of diagnosis and evaluation. Target cancer preferably includes urinary cancer. Examples of urinary cancer include tumors in the renal parenchyma, the urinary tract including the renal pelvis, the ureter, the bladder, and the urethra, and the genitals such as the prostate, the seminal vesicle, the penis, and the testicular, and representative examples include urothelial carcinoma, prostate cancer, kidney cancer, and testicular cancer.

The target cancer may include urothelial carcinoma. Examples of urothelial carcinoma include bladder cancer, ureteral cancer, urethral cancer, and renal pelvis cancer. The target cancer may be selected from the group consisting of bladder cancer, ureteral cancer, urethral cancer, and renal pelvis cancer.

Urothelial carcinoma refers to cancer occurring in a cell of the mucosal urinary tract designated as urothelium (transitional epithelium) present inside the urinary tract extending from the renal pelvis to the ureter, the bladder, and a part of the urethra. Principal one of urothelial carcinomas is bladder cancer, and the tumor grade of bladder cancer is evaluated in two stages of low grade and high grade, or in three stages of G1 to G3, and the tumor grade is higher and malignancy is higher in high grade and G3. Tumor depth (T stage) is classified as described below, and Ta to T1 are designated as non-muscle-invasive cancer, among which Tis is designated as carcinoma in situ, and T2 and higher stages corresponding to muscle-invasive cancer ("General Rule for Clinical and Pathological Studies on Renal Pelvis, Ureteral and Bladder Cancer), April 2011 (first edition)" edited by The Japanese Urological Association, The Japanese Society of Pathology, and Japan Radiological Society (Kanehara & Co., Ltd.)).
TX: Primary tumor cannot be assessed
T0: No evidence of primary tumor
Ta: Non-invasive papillary cancer
Tis: Carcinoma in situ (CIS)
T1: Tumor invading subepithelial connective tissue
T2a: Invasion into central portion of muscle
T2b: Invasion beyond central portion of muscle
T3a: Microscopic invasion into adipose tissue around bladder presumed
T3b: Macroscopically definite extramural invasion into adipose tissue around bladder presumed
T4a: Invasion into prostate, seminal vesicle, uterine, or vagina
T4b: Invasion into pelvic wall or abdominal wall

Currently, Nuclear matrix protein 22 (NMP22), Bladder tumor antigen test (BTA test) and the like are used as cancer biomarkers, but there is a problem that all of these markers have low sensitivity as a bladder cancer marker (particularly sensitivity to low grade cancer). On the contrary, a marker utilizing active PKCα in urine of the present invention exhibits a high specificity against and a high sensitivity to also non-muscle-invasive cancer, particularly low grade cancer. Accordingly, the target cancer may be, for example, non-muscle-invasive cancer, may be low grade cancer, and may be carcinoma in situ.

### [Method for Diagnosing Cancer]

A method of the present embodiment provides an index for diagnosing that a subject (examinee) has cancer based on that active PKCα is present in urine of a cancer patient. The method for diagnosing cancer of the present embodiment includes a step of detecting active PKCα in urine.

The method of the present embodiment may further include a step of collecting urine (urine specimen) corresponding to a test sample. Urine can be collected, for example, with a urine collecting container. Urine collected from a subject can be used. The subject may be a human, or may be a non-human mammal. The subject may be an animal suspected to have cancer.

The method of the present embodiment may include a step of appropriately pretreating the urine specimen in accordance with a method for detecting active PKCα. Examples of such a pretreatment include centrifugation, heating, addition of a surfactant, cell disruption with a homogenizer or an ultrasonic cell disruptor, dilution with a sample buffer or the like, and a treatment with a protease inhibitor.

The method of the present embodiment can provide an index for diagnosing cancer, for example, based on an amount of active PKCα present in urine. Examples of the index for diagnosing cancer include an index of having onset of cancer or a high possibility of future onset, and on the contrary, an index of having a low possibility of onset of cancer, or a low possibility of future onset. For example, that an amount of active PKCα in urine of a subject is larger than a reference value may indicate that the subject has cancer or has a high possibility of future onset. The reference value may be a value set based on the amount of active PKCα in urine of a healthy person, may be a value set based on the amount of active PKCα in urine of a cancer patient, or may be a value set by comparison between the amount of active PKCα in urine of a healthy person and the amount of active PKCα in urine of a cancer patient. The reference value may be, for example, a value for directly comparing the amount of active PKCα of the subject, or may be a value set as a factor (for example, 10-fold) in comparison with the amount of active PKCα in urine of a healthy person. The reference value may be a value set based on a plurality of samples, such as an average. Here, the term "healthy person" used in the above description may be replaced with a person not having cancer (non-cancer patient), and a non-cancer patient may be a person having been diagnosed not to have specific cancer (such as urinary cancer).

In addition, the method of the present embodiment can be used for determining a state (disease condition) of cancer by periodically monitoring the amount of active PKCα of one subject. For example, an increase trend of the amount of active PKCα in urine of a subject may indicate onset of cancer or a high possibility of future onset.

A method for detecting active PKCα in urine of a subject is not especially limited, and can be performed by a known method. For example, since active PKCα has a phosphorylation function, urine of a subject can be reacted with a substrate peptide of active PKCα to detect the peptide having been phosphorylated. Alternatively, for example, an antibody to active PKCα is reacted with urine to directly detect active PKCα. Accordingly, the step of detecting active PKCα in urine may include, for example, reacting a substrate peptide of active PKCα with the urine to detect the peptide having been phosphorylated, or may include reacting an antibody to active PKCα with the urine to detect PKCα.

### [Detection with Substrate Peptide]

When the step of detecting active PKCα in urine includes reacting a substrate peptide of active PKCα with the urine to detect the peptide having been phosphorylated, that the detected amount of the phosphorylated PKCα substrate peptide is larger than a reference value may indicate that the subject has cancer. The reference value may be, for example, set based on an amount of phosphorylated PKCα substrate peptide detected in urine of a healthy person, may be a value set based on an amount of phosphorylated PKCα substrate peptide detected in urine of a cancer patient, or may be a value set by comparison between the amount of phosphorylated PKCα substrate peptide detected in urine of a healthy person and the amount of phosphorylated PKCα substrate peptide detected in urine of a cancer patient. The reference value may be, for example, a value for directly comparing the amount of phosphorylated PKCα substrate peptide detected in a subject, or may be a value set as a factor (for example, 10-fold) in comparison with the amount of phosphorylated PKCα substrate peptide detected in urine of a healthy person. The reference value may be a value set based on a plurality of samples, such as an average. When the amount of phosphorylated PKCα substrate peptide is quantitatively determined by obtaining a phosphorylation percentage to the amount of PKCα substrate peptide used in the reaction as described below, the phosphorylation percentage may be used as the reference value. The phosphorylation percentage used as the reference value may be set, for example, in a range of 1 to 7%, 1.5% to 5%, 1.5 to 4%, or 2% to 3%.

The substrate peptide of active PKCα is not limited but may be any peptide specifically phosphorylated by PKCα, and an example includes a substrate peptide consisting of 10 to 30 amino acid residues (preferably 10 to 20 amino acid residues, and more preferably 10 to 15 amino acid residues). Specific examples of the peptide preferably include a peptide consisting of an amino acid sequence (FKKQGSFAKKK) of SEQ ID NO: 1, and a peptide consisting of an amino acid sequence (FKKQGTFAKKK) of SEQ ID NO: 2. It is noted that the peptide consisting of the amino acid sequence of SEQ ID NO: 1 is herein designated also as "Alphatomega".

The PKCα substrate peptide can be synthesized, for example, with an automated peptide synthesizer in accordance with standard Fmoc chemistry procedures. After the synthesis, the resultant can be purified by a known purification method using various chromatography or the like.

In the method of the present embodiment, the amount of the PKCα substrate peptide to be used can be appropriately set by those skilled in the art, and may be, for example, 1 to 1000 µM (preferably 1 to 500 µM, and more preferably 1 to 50 µM).

### (i) Detection of Phosphorylated PKCα Substrate Peptide

Urine collected as a test sample may be subjected to an appropriate pretreatment such as centrifugation, heating, addition of a surfactant, cell disruption with a homogenizer or an ultrasonic cell disruptor, dilution with a sample buffer or the like, or a treatment with a protease inhibitor. When the dilution is performed, as the sample buffer, for example, HEPES (4-(2-hydroxyethyl)-1-piperazineethanesulfonic acid) buffer, phosphate buffered saline, or the like can be used.

After performing the pretreatment if necessary, the test sample is contacted with the PKCα substrate peptide, and when active PKCα is present in the test sample, the PKCα substrate peptide is phosphorylated by its phosphorylation function. In the phosphorylation reaction, a reaction medium is not especially limited, and for example, a buffer obtained by mixing Tris-HCl and magnesium chloride or the like can be used, and as a phosphate donating substrate, for example, a mixture of adenosine triphosphate (ATP) or the like with the buffer can be used.

The reaction between the test sample and the PKCα substrate peptide is performed at 30 to 40°C (preferably 37°C) for 10 to 60 minutes (preferably 30 to 60 minutes).

The detection of the phosphorylated PKCα substrate peptide can be performed, although not limited thereto, by employing known immunoassay in accordance with the ordinary method. Examples of the immunoassay include labeled immunoassay and turbidimetric immunoassay (TIA), and examples include enzyme immunoassay (EIA) such as ELISA, radioimmunoassay (RIA) and fluoroimmunoassay (FIA). Examples of the labeled immunoassay combined with another separation method include Western blotting (combination with electrophoresis). Among these methods, ELISA and/or Western blotting is preferred. Alternatively, one or two or more monoclonal antibodies to the phosphorylated PKCα substrate peptide may be used to perform the detection with a combination of two or more immunoassays. Thus, excellent detection with further higher sensitivity and reliability can be performed.

As the method for detecting phosphorylated PKCα substrate peptide, a method using a metal colloid such as a metal nanoparticle can be also employed. Specifically, this method is a method based on principle that when a substrate peptide and protein kinase are reacted with each other in the presence of a metal colloid, or when a metal colloid is added after a reaction between a substrate peptide and protein kinase, the level of phosphorylation of the substrate peptide by protein kinase can be evaluated based on color change of the metal colloid. Regarding this color change, when a metal colloid is caused to present in the phosphorylation reaction of the substrate peptide by protein kinase, the phosphorylated substrate peptide is agglomerated due to the presence of the metal colloid particle, and the color of the metal colloid is changed based on the degree of agglomeration. The color of the metal colloid changes in accordance with the degree of agglomeration substantially in proportion to phosphorylation of the substrate peptide.

Accordingly, a reaction between the test sample and the PKCα substrate peptide is performed in the presence of a metal colloid, or the metal colloid is added after the reaction, and color change of the sample is compared with that of the sample before the reaction, or compared with color change of a control (reaction with a sample collected from a healthy person), and thus, the presence of phosphorylation of the PKCα substrate peptide can be detected.

In the method using the metal colloid, as a reaction medium, for example, a buffer obtained by mixing HEPES (4-(2-hydroxyethyl)-1-piperazineethansulfonic acid) and magnesium chloride, or the like is used, and as a phosphate donating substrate, ATP or the like is used in the same manner as described above.

As the metal colloid of metal nanoparticles or the like, for example, a gold colloid, a silver colloid, a platinum colloid, an iron colloid, an iron hydroxide colloid, an aluminum hydroxide colloid, a palladium colloid, a rhodium colloid or the like is used. Among these metal colloids, a gold colloid and a silver colloid are preferred, and a gold colloid is more preferred. These metal colloids can be produced by methods described in known literatures, or a commercially available product can be used. For example, a gold nanoparticle can be generally produced by a liquid phase reduction method in which a reducing solution is added to a solution containing a gold ion to reduce the gold ion (see, for example, Japanese Unexamined Patent Publication No. 2003-253310, and Japanese Unexamined Patent Publication No. 2006-152438).

A particle size of the metal colloid is not especially limited, and for example, is preferably about 1 nm to 500 nm, more preferably 3 nm to 200 nm, and further preferably 5 nm to 100 nm.

Although the color of a metal colloid depends on the particle size, a gold colloid is usually red, and a silver colloid is yellow. On the contrary, for example, when a gold colloid is present, phosphorylation and agglomeration of the PKCα substrate peptide results in changing the color of the gold colloid from red to blue. Based on such color change, the phosphorylated PKCα substrate peptide can be detected. It is noted that the color change of a metal colloid is checked by measuring an absorbance preferably in a range of about 600 nm to 800 nm, more preferably in a range of about 600 nm to 750 nm, and further preferably at about 700 nm.

### (ii) Quantitative Determination of Phosphorylated PKCα Substrate Peptide

Although not limited thereto, quantitative determination can be performed by obtaining a phosphorylation percentage to PKCα substrate peptide used in the reaction (ionic strength ratio between a phosphorylated substrate and a non-phosphorylated substrate) by employing matrix-assisted laser desorption/ionization time-of-flight mass spectrometry (MALDI-TOF MS).

In addition, when the phosphorylated PKCα substrate peptide is detected by the method using the metal colloid, the phosphorylated substrate peptide can be substantially quantitatively determined based on the degree of color change of the metal colloid (specifically, by comparing the degree of color change obtained with an appropriate control, or with a precedently created calibration curve).

The quantitative determination or detection of the phosphorylated PKCα substrate peptide can be performed with a commercially available kit.

### [Detection with Antibody]

When the step of detecting active PKCα in urine includes reacting an antibody to active PKCα with the urine to detect the PKCα, that a detected amount of active PKCα is larger than a reference value may indicate that the subject has cancer. The reference value may be a value set based on an amount of active PKCα detected in urine of a healthy person, may be a value set based on an amount of active PKCα detected in urine of a cancer patient, or may be a value set by comparison between an amount of active PKCα detected in urine of a healthy person and an amount of active PKCα detected in urine of a cancer patient. The reference value may be, for example, a value for directly comparing the detected amount of active PKCα of the subject, or may be a value set as a factor (for example, 10-fold) in comparison with the amount of active PKCα detected in urine of a healthy person. The reference value may be a value set based on a plurality of samples, such as an average.

An antibody to active PKCα (anti-PKCa antibody) can be produced as follows.

### (A) Production of Polyclonal Antibody

### (i) Preparation of Antigen and Solution Thereof

For producing an anti-PKCa antibody, it is first necessary to prepare or obtain a protein to be used as an immunogen (antigen).

An antigen protein can be, but is not limited to, purified PKCα, and for example, a protein having an amino acid sequence obtained by deleting, substituting or adding one or several amino acids in the amino acid sequence of PKCα, and having PKCα activity can be used. It is noted that, for example, PKCα derived from a mammal, specifically, human-derived PKCα, mouse-derived PKCα, or rat-derived PKCα, can be used as the PKCα. Here, nucleotide sequence and amino acid sequence information of PKCα derived from each animal is available as the following Accession Nos.

### <Human-derived PKCα>

- GenBank Accession No: NM 002737 (nucleotide sequence: SEQ ID NO: 3), NP 002728 (amino acid sequence: SEQ ID NO: 4)
- GenBank Accession No: X52479 (nucleotide sequence: SEQ ID NO: 5), CAA36718 (amino acid sequence: SEQ ID NO: 6)

### <Mouse-derived PKCα>

- GenBank Accession No: NM 011101 (nucleotide sequence: SEQ ID NO: 7), NP 035231 (amino acid sequence: SEQ ID NO: 8)
- GenBank Accession No: M25811 (nucleotide sequence: SEQ ID NO: 9), AAA39934 (amino acid sequence: SEQ ID NO: 10)

### <Rat-derived PKCα>

- GenBank Accession No: NM 001105713.1 (nucleotide sequence: SEQ ID NO: 11), XM 343975 (nucleotide sequence: SEQ ID NO: 12)

A method for preparing purified PKCα to be used as an antigen is not limited, and for example, a method in which PKCα is produced using a transformant of an appropriate host cell (such as a human-derived cultured fibroblast, a Chinese hamster ovary cell, or yeast) for preparation by a general biochemical method employed for isolation purification of a protein, for example, one of or a combination of ammonium sulfate precipitation, gel chromatography, ion exchange chromatography, and affinity chromatography can be employed.

Next, the purified PKCα thus obtained is dissolved in a buffer to prepare an antigen solution. At this point, an adjuvant for effective immunization may be added if necessary. As the adjuvant, for example, commercially available Freund's complete adjuvants or Freund's incomplete adjuvants can be used. One of such adjuvants may be used, or two or more of these may be used together, which is not restrictive.

### (ii) Immunization, and Collection of Anti-serum

Immunization is performed by administering a solution containing the purified PKCα to a mammal (such as a mouse, a rat, and a rabbit). The administration is performed mainly by intravenous, subcutaneous, or intraperitoneal injection.

A single dose of the antigen solution is not limited, and for example, preferably contains the purified PKCα of 2 to 500 µg, and more preferably 10 to 100 µg per animal.

A dosage interval is not limited, and for example, is preferably an interval of several days to several weeks, and more preferably an interval of 2 to 3 weeks. The number of doses is, for example, 2 to 10.

Although not limited thereto, a serum obtained by the immunization (anti-serum) is collected, for example, preferably 1 to 28 days, and more preferably 2 to 14 days after a day of the final administration. As a method for collecting an anti-serum, the anti-serum can be collected from blood of the immunized animal by an ordinary method.

### (iii) Sorting of Intended Anti-serum

From the anti-serums collected from respective immunized animals, an intended anti-serum, namely, an anti-serum containing an anti-PKCa antibody, is screened.

A screening method is not limited, and screening can be performed, for example, by employing any of known immunoassays in accordance with an ordinary method using the collected anti-serum, and the purified PKCα used as the antigen or recombinant PKCα (produced in a human-derived cultured fibroblast, a Chinese hamster ovary cell, or yeast), or a mutant enzyme thereof. Examples of the immunoassays include labeled immunoassay and turbidimetric immunoassay (TIA), and the former is preferred, and preferable examples include enzyme immunoassay (EIA) such as ELISA, radioimmunoassay (RIA) and fluoroimmunoassay (FIA). As the labeled immunoassay, preferable examples of its combination with another separation method include Western blotting (combination with electrophoresis). It is noted that since a protein sample to be detected is denatured with heat and a surfactant in Western blotting, an antigen to be used in the screening method is a mutant enzyme.

The anti-PKCa antibody contained in the intended anti-serum obtained by the screening is usually a polyclonal antibody. When the antibody needs to be purified, purification can be performed by appropriately selecting, for example, only one of, or a combination of two or more of known purification methods, such as ammonium sulfate salting-out method, ion exchange chromatography, affinity chromatography, and gel chromatography.

A reactive site in PKCα to which the anti-PKCa antibody can specifically bind (specifically recognize) is not especially limited.

### (B) Production of Monoclonal Antibody

### (i) Preparation of Antigen and Solution Thereof

Preparation of an antigen and a solution thereof can be performed in the same manner as in the production of the polyclonal antibody described above.

### (ii) Immunization, and Collection of Antibody-Producing Cell

An immunization method, as well as a dose, a dosage interval, and the number of doses using the antigen solution can adopt the same method and conditions employed in the production of the polyclonal antibody described above.

Although not limited thereto, a cell producing the anti-PKCa antibody (antibody-producing cell) obtained by the immunization is collected, for example, preferably 1 to 14 days, and more preferably 2 to 4 days after a day of the final administration.

Preferable examples of the antibody-producing cell include a spleen cell, a lymph node cell (particularly, a regional lymph node cell), and a peripheral blood cell, and in particular, a spleen cell and a regional lymph node cell (such as a popliteal lymph node cell) are more preferred.

### (iii) Cell Fusion

Cell fusion of the thus collected antibody-producing cell and a myeloma cell is performed, and thus, a fused cell (hybridoma) can be obtained.

As the myeloma cell, an established cell generally available in a mammal such as a mouse can be used. In more detail, an established cell that has drug selectivity, and has a property that it cannot grow in a HAT selective medium (hypoxanthine-, aminopterin- and thymine-containing medium) in an unfused state but can grow in a state fused with the antibody-producing cell is preferred. Specifically, as a mouse myeloma cell, for example, PAI, P3X63-Ag.8.U1 (P3U1), NS-I and the like can be used.

The cell fusion is performed, for example, by mixing the antibody-producing cell and the myeloma cell and causing a fusion reaction therebetween in an animal cell culture medium such as serum-free RPMI-1640 medium. A mixing ratio between the antibody-producing cell and the myeloma cell is, for example, 5:1.

The fusion reaction is performed preferably in the presence of a cell fusion promoter in general, and as the promoter, polyethylene glycol having an average molecular weight of 1000 to 6000 Dalton, or the like can be used. Alternatively, a commercially available cell fusion device utilizing electrical stimulation (such as electroporation) can be used for fusing the antibody-producing cell and the myeloma cell.

The cell resulting from the fusion reaction is cultured in, for example, HAT selective medium. After the culture, a cell found to grow in the HAT selective medium corresponds to the fused cell (hybridoma).

### (iv) Sorting of Intended Hybridoma, and Cloning thereof

From hybridomas obtained through the above-described culture, an intended hybridoma, namely, a hybridoma producing the anti-PKCa antibody is screened. Specifically, a culture supernatant containing the intended anti-PKCa antibody is screened.

A screening method is not limited, and for example, the screening can be performed by employing any of known immunoassays in accordance with an ordinary method using a part of the culture supernatant, and the purified PKCα used as the antigen or recombinant PKCα (produced in a human-derived cultured fibroblast, a Chinese hamster ovary cell, or yeast), or a mutant enzyme thereof. Examples of the immunoassays include enzyme immunoassay (EIA) such as ELISA, radioimmunoassay (RIA) and fluoroimmunoassay (FIA). Western blotting (combination with electrophoresis) or the like is also preferably employed. It is noted that since a protein sample to be detected is denatured with heat and a surfactant in Western blotting, an antigen to be used in the screening method is a mutant enzyme.

The anti-PKCa antibody contained in a culture supernatant of the intended hybridoma resulting from the screening is the antibody obtained before cloning the hybridoma, and may be an antibody containing a single molecule (monoclonal antibody), which is not restrictive.

A reactive site in PKCα to which the anti-PKCa antibody can specifically bind (specifically recognize) is not especially limited, and for example, it may be an antibody specifically recognizing a site where PKCα is phosphorylated (such as sites of Thr497, Thr638, and Ser657). When an antibody specifically recognizing a site where PKCαa is phosphorylated is used, detection accuracy of active PKCα can be expected to be improved.

Cloning of the intended hybridoma by the screening, namely, establishment of a monoclonal antibody-producing cell line, can be performed generally by selecting a colony derived from a single cell through culture by a limiting dilution method or the like.

### (v) Collection of Monoclonal Antibody

A method for collecting the monoclonal antibody from the hybridoma resulting from the cloning is not limited, and a cell culture method, or an ascites formation method or the like can be generally employed.

In the cell culture method, the hybridoma resulting from the cloning is cultured, for example, in an animal cell culture medium under conditions of 37°C and 5% CO₂ for 7 to 14 days, and the intended monoclonal antibody can be collected from a culture supernatant thus obtained.

In the ascites formation method, for example, the hybridoma resulting from the cloning is administered, in an amount of 10⁶ cells per animal, intraperitoneally to an animal of the same species as the mammal from which the myeloma cell used in the cell fusion is derived, and thus, the hybridoma is grown in a large amount, and the intended monoclonal antibody can be collected from ascites fluid or serum collected 7 to 14 days after the administration.

In employing either of the cell culture method and the ascites formation method, when the monoclonal antibody needs to be purified in or after collecting the antibody, only one of, or a combination of two or more of known purification methods, such as ammonium sulfate salting-out method, ion exchange chromatography, affinity chromatography, and gel chromatography can be appropriately selected and performed.

In the method of the present embodiment, an amount of the anti-PKCa antibody to be used can be appropriately set by those skilled in the art, and may be, for example, with 1 µg assumed as a standard, 1 µg to 10 µg, 1 µg to 100 µg or 1 µg to 1000 µg.

### (C) Detection and Quantitative Determination of Active PKCα

### (i) Detection of active PKCα

Urine collected as a test sample may be subjected to an appropriate pretreatment such as centrifugation, heating, addition of a surfactant, cell disruption with a homogenizer or an ultrasonic cell disruptor, dilution with a sample buffer or the like, or a treatment with a protease inhibitor. When the dilution is performed, as the sample buffer, for example, HEPES (4-(2-hydroxyethyl)-1-piperazineethanesulfonic acid) buffer, phosphate buffered saline, or the like can be used.

After performing the pretreatment if necessary, the test sample is reacted with the anti-PKCa antibody (preferably a monoclonal antibody in general), and when the test sample contains active PKCα, an antigen-antibody reaction occurs.

The reaction between the test sample and the anti-PKCa antibody is performed at 20 to 40°C (preferably 30 to 37°) for 30 to 720 minutes (preferably 30 to 90 minutes).

The detection of active PKCα can be performed by employing any of known immunoassays in accordance with an ordinary method thereof. Examples of the immunoassays include labeled immunoassay and turbidimetric immunoassay (TIA), and the former is preferred, and preferable examples include enzyme immunoassay (EIA) such as ELISA, radioimmunoassay (RIA) and fluoroimmunoassay (FIA). As the labeled immunoassay, preferable examples of its combination with another separation method include Western blotting (combination with electrophoresis). Among these methods, ELISA and/or Western blotting is preferred. In addition, one or two or more anti-PKCa monoclonal antibodies may be used, and the detection may be performed by a combination of two or more immunoassays. Thus, excellent detection with still higher sensitivity and reliability can be performed.

### (ii) Quantitative Determination of Active PKCα

Although quantitative determination is not limited, active PKCα contained in a test sample is quantitatively determined preferably from a calibration curve based on the relationship between an antigen level (antigen concentration: PKCα concentration) and a detected value thereof. The calibration curve is precedently created based on detected values (detection data) obtained by using purified PKCα as the antigen and using the anti-PKCa antibody. In more detail, information of detected values against the antigen concentration is obtained by a method similar to the immunoassay (such as ELISA or Western blotting) described above as the detection method, and the calibration curve is created based on the information. When the calibration curve is compared with an actually detected value (measured value), an amount of active PKCα in the test sample can be obtained. The quantitative determination or detection of PKCα can be performed with a commercially available kit.

### [Cancer Diagnosis Composition]

A cancer diagnosis composition of the present embodiment contains an antibody or a substrate peptide for detecting active PKCα, and is characterized by that diagnosis is made using urine of an examinee.

As the antibody or the substrate peptide for detecting active PKCα, those described above can be used. The cancer diagnosis composition of the present embodiment is used for detecting and quantitatively determining active PKCα in urine of an examinee, and can be used for the diagnosis described above. A method for detecting active PKCα and the like are the same as those described above.

A compounding ratio of the antibody in the cancer diagnosis composition is not especially limited, and for example, may be 1 to 100% by weight, 5 to 100% by weight, 10 to 100% by weight, 3 to 99% by weight, 5 to 99% by weight, 10 to 99% by weight, 10 to 95% by weight, or 20 to 95% by weight based on the total weight of the composition. Although not limited thereto, the cancer diagnosis composition of the present embodiment may contain, in addition to the anti-PKCa antibody, another constituent component as long as the effects of the present invention are largely impaired. For example, a primary antibody detection reagent, a chromogenic substrate and the like can be contained.

A compounding ratio of the PKCα substrate peptide in the cancer diagnosis composition is not especially limited, and for example, may be 1 to 100% by weight, 5 to 100% by weight, 10 to 100% by weight, 3 to 99% by weight, 5 to 99% by weight, 10 to 99% by weight, 10 to 95% by weight, or 20 to 95% by weight based on the total weight of the composition. Although not limited thereto, the cancer diagnosis composition of the present embodiment may contain, in addition to the PKCα substrate peptide, another constituent component as long as the effects of the present invention are largely impaired. For example, an antibody to the phosphorylated PKCα substrate peptide or the like can be contained.

### [Cancer Diagnosis Kit]

A cancer diagnosis kit of the present embodiment includes an antibody or a substrate peptide for detecting active PKCα, and is characterized by that diagnosis is made using urine of an examinee.

As the antibody or the substrate peptide for detecting active PKCα, those described above can be used. The cancer diagnosis kit of the present embodiment is used for detecting and quantitatively determining active PKCα in urine of an examinee, and can be used for the diagnosis described above. A method for detecting active PKCα and the like are the same as those described above.

The cancer diagnosis kit of the present embodiment may include the cancer diagnosis composition described above.

When the cancer diagnosis kit includes the anti-PKCa antibody, the antibody is preferably provided in a dissolved state in consideration of stability (preservability), ease of use, and the like.

When the cancer diagnosis kit includes the PKCα substrate peptide, the substrate peptide is preferably provided in a state of a power or in a state of a solution (state dissolved in pure water, a buffer or a saline) and stored under conditions of cold storage or frozen storage (-30°C to -80°C) in consideration of stability (preservability), ease of use, and the like.

The kit can include, in addition to the PKCα substrate peptide or the anti-PKCa antibody, another constituent element. Examples of another constituent element include a primary antibody detection reagent, a chromogenic substrate or the like that can be used in ELISA or Western blotting.

The kit can be any kit including, as a constituent element, at least the PKCα substrate peptide or the anti-PKCa antibody. Accordingly, all the constituent elements essential for cancer diagnosis may be, or may not be included in addition to the PKCα substrate peptide or the anti-PKCa antibody, which is not restrictive.

The kit may include means for collecting urine, such as a urine collecting container.

The kit may be provided with instructions for use of the kit to detect active PKCα in urine of an examinee for cancer diagnosis.

### [Method for Evaluating State of Cancer]

In a method for evaluating a state of cancer of the present embodiment, active PKCα is detected by reacting an antibody to active PKCα with urine of an examinee, and a state of cancer is evaluated by using, as an index, a detection result thus obtained. Further, in a method for evaluating a state of cancer of another embodiment, a substrate peptide of active PKCα is reacted with urine of an examinee to detect the peptide having been phosphorylated, and a state of cancer is evaluated by using, as an index, a detection result thus obtained.

As the antibody or the substrate peptide for detecting active PKCα, those described above can be used. A detection method by reaction with urine of an examinee is also the same as that described above.

By the method for evaluating a state of cancer of the present embodiment, a state of cancer can be evaluated by using, as an index, a detection result (detected amount) of active PKCα or the phosphorylated substrate peptide detected or quantitatively determined. In other words, by detecting active PKCα by reacting an antibody to active PKCα with urine of an examinee, or by detecting a phosphorylated substrate peptide by reacting the PKCα substrate peptide with urine, a state of cancer is evaluated by using, as an index, a detection result thus obtained.

Here, to "evaluate a state of cancer" means that onset, progression, severity, response to treatment, prognosis or the like of cancer is determined. The evaluation may be performed based on a detected amount of active PKCα or a detected amount of phosphorylated PKCα substrate peptide, and in addition, subjective symptom and the like may be used in combination. For example, the evaluation can be performed by measuring active PKCα or phosphorylated PKCα substrate peptide at a frequency of once or twice a year as in routine medical examination, but it is preferable to comprehensively evaluate a state of cancer (including monitoring of recurrence of cancer) by periodically monitoring transition of the detected amount of active PKCα or the phosphorylated substrate peptide. As such an evaluation method, it is possible, for example, to diagnose that there is a high possibility of onset of any cancer when the detected amount of active PKCα or the phosphorylated PKCα substrate peptide is over a prescribed value, to diagnose that treatment is effective when the detected amount of active PKCα or the phosphorylated substrate peptide is reduced in a cancer case under treatment, or to diagnose that treatment and recovery are impossible in a case where the detected amount of active PKCα or the phosphorylated substrate peptide is over a prescribed value and averaged at a high value.

### [Method for Screening Agents for Preventing and/or Treating Cancer]

A method for screening agents for preventing and/or treating cancer of the present embodiment includes: (a) a step of administering a candidate substance to a urinary cancer model non-human mammal (administration step); (b) a step of detecting active PKCα in urine of the non-human mammal (detection step); and (c) a step of selecting an intended agent for preventing and/or treating cancer with a result thus obtained used as an index (evaluation step). It is noted that the screening method of the present embodiment may include another step if necessary.

### These steps will now be described.

### (a) Administration Step

A urinary cancer model non-human mammal used as a subject animal, and a control animal are not especially limited, and from the viewpoint that an accurate comparative experiment can be performed, it is usually preferable to use non-human mammals of the same species, more preferable to use a litter of non-human mammals, and further preferable to use non-human mammals of the same sex and the same age. The subject animal and the control animal are preferably raised under the same conditions except for feed intake.

As the subject animal, a urinary cancer model non-human mammal obtained by causing cancer in a normal non-human mammal by transplantation of a tumor tissue or tumor cell by a known method can be used. Furthermore, a non-human mammal obtained by surgically resecting the tumor tissue or tumor cell from the urinary cancer model non-human mammal can be used. When the non-human mammal obtained after resection is used as the subject animal, for example, a method for screening agents for preventing recurrent cancer can be performed.

The control animal is not limited as long as it is suitable to be used as a comparative control of the subject animal, and for example, a normal non-human mammal in which a tumor tissue or the like is not transplanted is used, or alternatively, a urinary cancer model non-human mammal to which a candidate substance is not administered, or the subject animal before administration of a candidate substance can be used as the control animal.

A candidate substance to be administered to the subject animal is not limited, and examples include natural or artificially synthesized various peptides, proteins (including enzymes and antibodies), nucleic acids (such as polynucleotides (DNA and RNA), oligonucleotides (such as siRNA), and peptide nucleic acids (PNA), and low molecular weight or high molecular weight organic compounds.

The administration of a candidate substance can be orally or parenterally performed, which is not restrictive, and in either case, any of known administration methods and administration conditions can be employed. The dose can be also appropriately set in consideration of the type and the condition of the subject animal, and the type and the like of the candidate substance.

### (b) Detection Step

In this step, active PKCα is detected in urine (urine sample) of the subject animal to which the candidate substance has been administered. As a method for collecting the urine sample, a known method can be applied. The number of times of detecting active PKCα, the period of the detection and the like are not especially limited, and can be appropriately set in consideration of efficacy and the like of an intended agent for preventing or treating cancer.

Methods for detecting and quantitatively determining PKCα in urine are not limited, and the above-described methods can be appropriately employed.

### (c) Evaluation Step

In screening an agent for preventing and/or treating cancer, the detected amount (detection level) of active PKCα in urine is evaluated by comparison between, for example, the subject animal to which the candidate substance has been administered, and the control animal to which the candidate substance has not been administered. It is preferable to select, through this evaluation, whether or not the candidate substance is an intended agent for preventing and/or treating cancer.

Specifically, in urinary cancer model non-human mammals having the amount of active PKCα in urine at the same level, one is used as a subject animal to which the candidate substance is administered, and the other is used as a control animal to which the candidate substance is not administered, and in this case, when the amount of active PKCα in urine of the subject animal is reduced to a level lower than the amount of active PKCα in urine of the control animal, the candidate substance can be selected as the intended agent for treating cancer. On the contrary, when the amount of active PKCα in urine of the subject animal is retained at the same level as or increased to a level higher than the amount of active PKCα in urine of the control animal, the candidate substance cannot be selected as the intended agent for treating cancer. In this case, the subject animal itself before the administration of the candidate substance can be a control animal.

Alternatively, in a case where a urinary cancer model non-human mammal in which a tumor tissue or the like has been surgically resected, or a urinary cancer model non-human mammal in which cancer treatment has been completed by a method except for the surgical resection (such as a medical method) is used as a subject animal to which the candidate substance is administered, and a normal non-human mammal is used as a control animal, when the amount of active PKCα in urine of the subject animal is continuously retained at the same level as the amount of PKCα in urine of the control animal, the candidate substance can be selected as an agent for preventing cancer (specifically, an agent for preventing recurrent cancer). On the contrary, when the amount of active PKCα in urine of the subject animal is increased to a level higher than the amount of PKCα in urine of the control animal, the candidate substance cannot be selected as an intended agent for preventing cancer. In this case, the subject animal itself immediately before the administration of the candidate substance (before the recurrence) can be used as the control animal.

It is noted that the screening of agents for preventing and/or treating cancer based on the amount of active PKCα in urine can be performed on a plurality of types of cancer (preferably all the types of cancer), and hence, if a urinary cancer model non-human mammal of a specific type of cancer is created and used as the urinary cancer model non-human mammal, agents for preventing and/or treating the specific type of cancer can be screened.

### [Method for Treating Cancer]

A method for treating cancer of the present embodiment includes: a cancer diagnosis step including detecting active PKCα in urine of an examinee; and a step of administering a therapeutic agent to the examinee when he/she is diagnosed to have cancer.

The cancer diagnosis step may be a step of diagnosing whether or not the examinee has cancer based on an index provided by the method for diagnosing cancer described above. In addition, the cancer diagnosis step may be a step of diagnosing whether or not the examinee has cancer based on the method for evaluating a state of cancer described above.

In the step of administering a therapeutic agent to the examinee when he/she is diagnosed to have cancer, the therapeutic agent to be administered to the examinee can be appropriately selected by those skilled in the art depending on the type of the cancer, and for example, when the cancer is urinary cancer, an anthracycline antitumor antibiotic, mitomycin, and BCG are usually used as a transurethral postoperative recurrence preventive agent, and cisplatin and gemcitabine are usually used as a therapeutic agent in many cases. A therapeutic agent selected by the method for screening agents for preventing and/or treating cancer described above can be used. The administration can be orally or parenterally performed, which is not restrictive, and in either case, any of known administration methods and administration conditions can be employed. The dose can be also appropriately set in consideration of the type of the cancer and the condition of the examinee, and the type and the like of the therapeutic agent.

The method for treating cancer of the present embodiment may further include a step of determining, after administering the therapeutic agent, whether or not treatment with the therapeutic agent is effective by monitoring a detected amount of active PKCα or the phosphorylated substrate peptide by the method for evaluating a state of cancer described above. For example, the method may include determining that the treatment is effective when the detected amount of active PKCα or the phosphorylated substrate peptide is reduced after the administration of the therapeutic agent, and/or determining that the treatment is not effective in a case where the detected amount of active PKCα or the phosphorylated substrate peptide is over a prescribed value and averaged at a high value.

### Examples

The present invention will now be more specifically described with reference to examples, and it is noted that the present invention is not limited to these examples.

### [Example 1 Phosphorylation of Substrate Peptide in Cancer Cell Sample]

### (Synthesis of Substrate Peptide)

Alphatomega (FKKQGSFAKKK) (SEQ ID NO: 1)) to be used as a substrate peptide was synthesized with an automated peptide synthesizer used in accordance with standard Fmoc chemistry procedures. After treatment with trifluoroacetic acid (TFA), the resultant peptide was purified on an Inertsil ODS-3 column (250 x 20 mm, 3.5 µm; GL Sciences Inc., Tokyo, Japan) with BioCAD perfusion chromatography system (Ikemoto Scientific Technology Co., Ltd.) and A-B linear gradient at a flow rate of 8 ml/min (eluent A: 0.1% TFA-containing water, eluent B: 0.1% TFA-containing acetonitrile).

This peptide was used in the following experiment as PKCα specific substrate peptide. A molecular weight of this substrate peptide FKKQGSFAKKK-NH₂ is 1338 Da, and is increased by 80 Da to 1418 Da through a phosphorylation reaction with PKCα.

### (MALDI-TOF MS Analysis)

An α-cyano-4-hydroxycinnamic acid (CHCA) matrix (10 mg/ml) was prepared in 50% water/acetonitrile and 0.1% TFA. This matrix and a sample were mixed in a ratio of 20:1. The resultant specimen/matrix mixture in a total volume of 1 µl was applied on a sample plate, and the resultant was crystallized by drying. In this example, Auto Flex Speed manufactured by Bruker was used in positive or negative mode. An acceleration voltage was set to 20 kV, and extraction delay time was set to 100 ns. Typically, 100 laser shots were averaged to improve a signal/noise ratio. All spectra were analyzed with Flex Analysis software (Bruker). A phosphorylation percentage was defined as an ionic strength ratio between a phosphorylated substance and a non-phosphorylated substance, and was calculated in accordance with existing description (Kang, J. -H. et al., (2007) J. Am. Soc. Mass Spectrom. 18, 106-112; and Kang J. -H. et al., (2007) J.Am. Soc. Mass Spectrom. 18, 1925-1931).

In order to examine whether or not the PKCα specific substrate peptide could be phosphorylated by bladder cancer, culture cells derived from 5 different bladder cancers (KU-1, KU-7, T24, TCCSUP, and UMUC-3) were analyzed by MALDI-TOF MS. KU-1, KU-7, TCCSUP, and UMUC-3 were maintained in Dulbecco's modified Eagle's media supplemented with 10% fetal bovine serum, penicillin (100 U/ml), streptomycin (100 µg/ml) and amphotericin B (0.25 µg/ml; all Invitrogen). T24 was maintained in RPMI-1640 medium. The resultant cancer cells were placed in a humidified atmosphere containing 5% CO₂ and 95% air. The resultant culture cells were detached, centrifuged at 1,500 rpm for 5 minutes, and the resultant sediment (cells) was used for a phosphorylation reaction with the substrate peptide. The phosphorylation of the substrate peptide was performed in 30 µl of buffer containing 30 µM synthetic peptide (10 mM HEPES (pH 7.5), 10 mM MgCl₂, 100 µM ATP, and 0.2 mg/ml cells). After performing incubation at 37°C for 60 minutes, the resultant sample was analyzed by MALDI-TOF MS.

Results are illustrated in Figures 1 and 2. "UNTREATED" means containing no cells. The phosphorylation of the substrate peptide was confirmed by the increase of 80 Da in the m/z value, and high phosphorylation percentages were obtained in all samples prepared from bladder cancer-derived culture cells.

### [Example 2 Detection of PKCα Activity in Human Urine Sample]

Urines of 28 urothelial carcinoma patients were collected, and used as samples to phosphorylate a substrate peptide. As a control group, urines of 24 non-urothelial carcinoma patients were used. Case information of the urothelial carcinoma patients and the non-urothelial carcinoma patients are illustrated respectively in Figures 3 and 4.

20 ml of a urine specimen of each patient was collected, and centrifuged (5000 g, 15 minutes) to collect a sediment. To the resultant, 160 µl of HEPES buffer (containing 10% of sucrose) and 40 µl of cOmplete protease inhibiter cocktail were added for resuspension. The resultant was irradiated with a probe ultrasonic cell disruptor (SONIFIER 250 manufactured by BRANSON) for 10 seconds on ice to disrupt cells. The resultant was centrifuged (5000 g, 15 minutes), a supernatant was collected, and quantitative determination of protein was performed to obtain a protein concentration. The phosphorylation of the substrate peptide was performed with a protein concentration of 0.2 mg/ml, ATP of 100 µM, MgCl₂ of 10 mM, and the substrate peptide of 30 µM. After performing incubation at 37°C for 60 minutes, the resultant sample was analyzed by MALDI-TOF MS. The analysis of the substrate peptide and by MALDI-TOF MS was performed in the same manner as in Example 1.

Phosphorylation percentages of the urothelial carcinoma patients and the non-urothelial carcinoma patients were measured to illustrate a histogram (Figure 5), a probability density function (Figure 6), and a cumulative distribution function (Figure 7). The urothelial carcinoma patients exhibited higher phosphorylation as compared with the non-urothelial carcinoma patients, and a phosphorylation percentage of 2% providing a large difference therebetween was set as a threshold.

Figure 8 illustrates the phosphorylation percentages of the substrate peptide in urine samples from 9 low grade urothelial carcinoma patients and 19 high grade urothelial carcinoma patients. Both the low grade and high grade urothelial carcinoma patients exhibited high phosphorylation as compared with the non-urothelial carcinoma patients.

### [Example 3 Comparison with Other Urothelial Carcinoma Markers]

A threshold of positive reaction was set to 2% based on the phosphorylation percentages of PKCα of the urothelial carcinoma patients and the non-urothelial carcinoma patients calculated in Example 2, and comparison was made with other existing urothelial carcinoma markers (urine cytology, NMP22 (Alere), BTA (Fujirebo Inc.), and CK18-8 (iDL Biotech)). The existing urothelial carcinoma markers were used in accordance with attached documents.

The PKCα threshold of urothelial carcinoma patients was set to 2%, and regarding the other urothelial carcinoma markers, thresholds were set to 16 ng/mgCr for CK18-8, and 12 U/ml for NMP22, and sensitivity, specificity and accuracy of each measurement method were examined. Since urine cytology and BTA are qualitative methods, these were examined by employing positive/negative determination. PKCα exhibited a sensitivity of 89.3%, a specificity of 83.3%, and an accuracy of 86.5%, which were all high. On the other hand, NMP22 exhibited a sensitivity of 50.0%, a specificity of 91.7%, and an accuracy of 69.2%, TBA exhibited a sensitivity of 39.4%, a specificity of 75.0%, and an accuracy of 55.8%, and CK18-8 exhibited a sensitivity of 75.0%, a specificity of 58.3%, and an accuracy of 67.3%, and thus, their specificities were high but sensitivities were low (Table 1).

**[Table 1]**

| | Sensitivity (Positive/Number of Samples) | | | Specificity (Negative/Number of Samples) | Accuracy |
|---|---|---|---|---|---|
| | all grades | low grade | high grade | | |
| PKC | 89.3% (25/28) | 88.9% (8/9) | 89.5% (17/19) | 83.3% (20/24) | 86.5% |
| Urine Cytology | 71.4% (20/28) | 22.2% (2/9) | 94.7% (18/19) | - | - |
| NMP22 | 50.0% (14/28) | 11.1% (1/9) | 68.4% (13/19) | 91.7% (22/24) | 69.2% |
| BTA | 39.3% (11/28) | 0.0% (0/9) | 57.9% (11/19) | 75.0% (18/24) | 55.8% |
| CK8-18 | 75.0% (21/28) | 44.4% (4/9) | 89.5% (17/19) | 58.3% (14/24) | 67.3% |

Figure 9 illustrates receiver operating characteristic (ROC) curves of diagnosis based on the phosphorylation percentage of the substrate peptide using urine samples from 9 low grade urothelial carcinoma patients and 19 high grade urothelial carcinoma patients. Active PKCα in urine exhibited high detection sensitivity and specificity not only in the high grade urothelial carcinoma but also in the low grade urothelial carcinoma.

## Claims

1. A method for diagnosing cancer comprising:
a step of detecting active protein kinase Cα in urine.

2. The method according to claim 1, wherein the cancer includes urinary cancer.

3. The method according to claim 1 or 2, wherein the cancer includes urothelial carcinoma.

4. The method according to any one of claims 1 to 3, wherein the cancer includes carcinoma in situ.

5. A cancer diagnosis composition comprising:
an antibody or a substrate peptide for detecting active protein kinase Cα,
wherein diagnosis is made using urine of an examinee.

6. A cancer diagnosis kit comprising:
an antibody or a substrate peptide for detecting active protein kinase Cα,
wherein diagnosis is made using urine of an examinee.

7. A method for evaluating a state of cancer,
wherein active protein kinase Cα is detected by reacting an antibody to the active protein kinase Cα with urine of an examinee, and a state of the cancer is evaluated with an obtained detection result used as an index.

8. A method for evaluating a state of cancer by using, as an index, a detection result obtained by reacting a substrate peptide of active protein kinase Cα with urine of an examinee, and detecting the peptide having been phosphorylated.

9. A method for screening agents for preventing and/or treating cancer comprising:
a step of administering a candidate substance to a urinary cancer model non-human mammal;
a step of detecting active protein kinase Cα in urine of the non-human mammal; and
a step of selecting an intended agent for preventing and/or treating cancer with an obtained result used as an index.
